# FASCICULE DE BREVET EUROPEEN

(11) **EP 2 651 245 B1**
(45) Date de publication et mention de la délivrance du brevet: **16.12.2015**
(21) Numéro de dépôt: 11794796.0
(22) Date de dépôt: 16.12.2011
(51) Int. Cl.: A23L 1/0524, A23L 1/29, A61P 1/04, A23L 1/054

(54) **COMPOSITION ANTI-REGURGITATION ET/OU ANTI-REFLUX GASTRO-OESOPHAGIEN, PREPARATION ET UTILISATIONS**
ZUSAMMENSETZUNG GEGEN REGURGITATION UND/ODER GASTROÖSOPHAGEALEN REFLUX, HERSTELLUNG UND VERWENDUNG
ANTI-REGURGITATION AND/OR ANTI-GASTROOESOPHAGEAL REFLUX COMPOSITION, PREPARATION AND USES

(30) Priorité: 17.12.2010 FR 1060691
(43) Date de publication de la demande: 23.10.2013
(73) Titulaire: United Pharmaceuticals S.A., 75008 Paris (FR)
(72) Inventeur: MARGOSSIAN, Jonathan Albert, F-75016 Paris (FR); PRADEAU, Nicolas, F-35190 Tinteniac (FR); FALLOURD, Yannick, F-35000 Rennes (FR)
(74) Mandataire: Starck-Loudes, Anne-Caroline
(86) Numéro de dépôt international: PCT/EP2011/073039
(87) Numéro de publication internationale: WO 2012/080462

(56) Documents cités:
- WO-A1-01/56406
- WO-A1-2010/046321
- FR-A1- 2 913 857
- US-A- 6 099 871
- US-A1- 2002 193 344
- US-A1- 2003 165 606
- US-A1- 2004 142 017
- US-A1- 2004 228 903
- US-A1- 2004 258 825
- US-A1- 2006 078 649
- DATABASE WPI Week 200027 Thomson Scientific, London, GB; AN 2000-315804 XP002670148, & RU 2 130 728 C1 (CHILD FOODSTUFFS RES INST) 27 mai 1999 (1999-05-27)

## Description

Le domaine de la présente invention est celui de la médecine et de la nutrition humaine, en particulier de la nutrition infantile.

L'invention concerne des compositions destinées à réduire, idéalement à éliminer, les phénomènes de régurgitation et/ou de reflux gastro-oesophagien (RGO) affectant un sujet ainsi que les procédés permettant de les obtenir. Des compositions, formules, préparations et laits infantiles anti-régurgitation et/ou anti-reflux gastro-oesophagien destinés à l'alimentation des nouveau-nés, nourrissons et enfants en bas âge sont plus particulièrement décrits.

Les régurgitations constituent un symptôme fréquemment observé chez les nouveau-nés et nourrissons résultant d'une augmentation de la pression abdominale par rapport à la pression thoracique. Elles surviennent souvent après les repas ou lors de phénomènes d'éructations. Ces régurgitations, qui peuvent également affecter l'adulte, et se distinguent, au sens médical du terme, des vomissements, sont sans conséquence sur le développement de l'enfant et relèvent surtout de l'inconfort.
Le mécanisme des régurgitations peut être expliqué de la manière suivante : le sphincter oesophagien inférieur est un muscle circulaire situé dans la partie inférieure de l'oesophage. Lors des repas, ce muscle se relâche de manière à faciliter la propulsion des aliments ingérés (bol alimentaire) dans l'estomac. La fonction principale de ce muscle est de prévenir le retour du contenu de l'estomac vers l'oesophage grâce à sa constriction tonique.
Chez le nourrisson, ce muscle peut être immature. Il ne peut donc pas toujours soutenir et compenser la pression exercée par le contenu gastrique. Il a alors tendance, pendant et après les repas, à se relâcher et à laisser, au cours des régurgitations, remonter de petits volumes de liquide gastrique dans le pharynx et vers la bouche, via l'oesophage.
Ce phénomène apparaît généralement dès les premières semaines de la vie de l'enfant. Les facteurs de développement physiologique qui contribuent à l'apparition de ces régurgitations disparaissent, le plus souvent spontanément, lorsque l'enfant atteint l'âge de 12 à 15 mois (Vandenplas Y., Belli D. et al, Current concepts and issues in the management of regurgitation of infants : a reappraisal, Acta Pediatr 85 : 531-534, 1996).

Les régurgitations sont souvent à l'origine de l'inquiétude des parents qui, de ce fait, n'hésitent pas à consulter un médecin. Les préoccupations varient, chez l'enfant, de la simple gêne, occasionnée par les renvois, jusqu'à la douleur (la paroi de l'oesophage étant irritée, cela donne lieu à des brûlures voire à des ulcérations de l'oesophage). Ces douleurs sont sources de pleurs et compliquent souvent l'endormissement de l'enfant.
Dans certains cas, un reflux excessivement fréquent et/ou prolongé peut être responsable de complications, définissant, quel que soit l'âge du sujet atteint, un reflux gastro-oesophagien (RGO) pathologique.
Chez le nourrisson et l'enfant en particulier, le reflux pathologique peut avoir des conséquences oesophagiennes et respiratoires plus atypiques : cassure de la courbe staturo-pondérale, rhino-pharyngo-bronchite récidivante, asthme et bronchiolite, anémie.
On estime que les régurgitations et reflux surviennent chez environ 75% des enfants de moins de un an.

Le traitement recommandé par la Société Européenne de Pédiatrie, Gastro-entérologie, Hépatologie et Nutrition (ESPGHAN) consiste en un épaississement du bol alimentaire. Il a en effet été prouvé par des tests cliniques, qu'une augmentation de la viscosité du contenu gastrique peut réduire de manière significative la fréquence et le volume des régurgitations.
Les thérapeutiques médicamenteuses (procinétiques, antisécrétoires, etc.) sont essentiellement réservées aux régurgitations récalcitrantes et surtout aux vrais reflux.

Des épaississants classiquement employés depuis de très nombreuses années comme les amidons précuits et/ou prégélatinisés, la farine de graine de caroube ou les pectines peuvent être ajoutés, lors de la préparation du biberon, au lait infantile.

Les amidons, en association avec des protéines, les caséines en particulier, qui floculent à pH acide, développent, au niveau de l'estomac, la viscosité recherchée du bol alimentaire qui permet ainsi d'éviter les régurgitations et reflux par effet de pesanteur. Le lait absorbé, à pH neutre dans le biberon, exerce toutefois un effet tampon sur l'acidité gastrique, effet responsable du ralentissement de l'épaississement recherché. L'efficacité des amidons est alors fonction de la capacité de l'estomac du sujet à acidifier son alimentation, c'est-à-dire à abaisser le pH de environ 7 à environ 3, le plus rapidement possible. Chez l'enfant, l'acidification requise peut nécessiter entre 30 et 60 minutes ou plus, durée pendant laquelle l'enfant n'est pas à l'abri des régurgitations et reflux dont on cherche à le protéger.

Contrairement aux amidons dont l'effet dépend du pH de l'estomac, la farine de graine de caroube (encore identifiée en tant que « gomme de caroube », « caroube native », « caroube standard » ou « caroube naturelle ») est en mesure d'épaissir à pH neutre. L'épaississement du lait dans le biberon présente toutefois l'inconvénient, non négligeable pour l'enfant, de rendre le lait difficile à boire par la tétine. L'épaississement du lait dans le biberon peut être en conséquence à l'origine de problèmes d'aérophagie.

Un médicament, connu depuis plusieurs décennies, vendu en pharmacie sous l'appellation Gelopectose®, est par ailleurs utilisé pour épaissir le contenu de l'estomac et des selles. Ce médicament, constitué de pectine, de cellulose microcristalline et de silice colloïdale hydratée, se présente sous la forme d'une poudre à verser dans un biberon de lait reconstitué très chaud (50 à 60 °C). Le mode d'emploi du médicament recommande d'agiter vigoureusement le biberon, environ 30 secondes, puis de laisser le mélange reposer plusieurs minutes jusqu'à obtention d'un gel et de la température souhaitée (environ 37°C).
Bien qu'utilisé depuis plusieurs décennies, cette technique n'est pas satisfaisante. Elle est en effet longue et nécessite un moyen de chauffage. En outre, le dosage de la poudre ajoutée (2 cuillères à café rases pour 90 ml d'eau avant reconstitution du lait) est souvent imprécis.

Le principal inconvénient rencontré avec ce médicament est le même que celui rencontré avec la farine de graine de caroube, et est lié à l'épaississement du mélange dans le biberon. Cet épaississement est en effet responsable de la formation de grumeaux qui, très fréquemment, bouchent la tétine. Par ailleurs, la texture du produit reconstitué évolue dans le temps et impose une consommation rapide de la préparation.

La démarche précédemment décrite consistant à ajouter un épaississant au biberon de lait s'avérant peu pratique, des laits infantiles pré-épaissis dits laits « anti-régurgitation » (AR) sont par ailleurs proposés depuis plusieurs années.

Ces formulations AR contiennent déjà un agent épaississant, choisi parmi les amidons et la gomme de caroube native, à l'origine de la viscosité du lait reconstitué et permettent de surmonter certains des inconvénients décrits ci-dessus.
La gomme de caroube native est une caroube qui présente une solubilité faible en milieu aqueux de l'ordre de 20% à une température comprise entre 10°C et 45°C (cf. demande de brevet FR 2 913 857). L'expression "solubilité en milieu aqueux à une température comprise entre 10°C et 45°C" signifie qu'à une température comprise entre 10°C et 45°C, en milieu aqueux, la gomme de caroube développe au moins 20% de la viscosité qu'elle aurait développée si elle avait été mise en solution à des températures supérieures à 80°C.
La caroube native et les amidons ne présentent ainsi pas, à la température de reconstitution du lait dans le biberon (qui se situe entre environ 30°C et 50°C), une solubilité satisfaisante en milieu aqueux. Une telle solubilité étant cependant nécessaire et préalable à toute augmentation homogène de viscosité, les épaississants au moins partiellement, voire complètement solubles à la température de reconstitution du biberon, sont préférentiellement utilisés par les fabricants de laits infantiles anti-régurgitation.

Afin de répondre à ce besoin, des ingrédients et additifs ont été développés tels que des amidons pré-cuits ou pré-gélatinisés ainsi que des caroubes dits «solubles à froid» dont une proportion importante du potentiel visqueux s'exprime déjà entre 30 et 50°C. La gomme de caroube "soluble à froid" est une caroube qui présente une solubilité en milieu aqueux supérieure à 60% à une température comprise entre 10°C et 45°C selon la définition donnée ci-dessus.

La capacité des caroubes « solubles à froid » à provoquer une augmentation rapide de la viscosité de la base de lait liquide rend toutefois compliqué, et en pratique impossible, l'application de certains traitements technologiques, tels qu'un traitement thermique (lors de l'étape de pasteurisation ou stérilisation), l'homogénéisation et l'atomisation (pulvérisation) nécessaires à la production de poudre alimentaire, en particulier de laits infantiles en poudre.

Il en est de même pour les amidons pré-cuits ou pré-gélatinisés dont la structure résiste mal aux contraintes de cisaillement importantes auxquelles la base de lait liquide est soumise lors des étapes d'homogénéisation et d'atomisation. L'éclatement de la structure de l'amidon entraîne une perte importante voir totale de la viscosité finale du lait AR reconstitué.

Les amidons, tout comme la caroube « soluble à froid », ne peuvent donc qu'être ajoutés par mélange à sec à la poudre de lait infantile.

Un problème majeur posé par le mélange à sec d'amidon et/ou de caroube soluble à froid avec les ingrédients constitutifs classiques d'une base de lait infantile concerne cependant l'homogénéité du produit fini. Les quantités relativement faibles de farine de caroube ou d'amidon pré-cuit et/ou pré-gélatinisé à ajouter à la base de lait infantile en poudre et les différences de propriétés physiques des deux poudres à mélanger, compliquent en effet notablement l'obtention d'un produit fini homogène.

Des procédés de préparation de lait infantile anti-régurgitation liquide à faible teneur en matières sèches (entre 11 et 15% en masse), impliquant un traitement « UHT » (Ultra Haute Température) ont par ailleurs été décrits (voir par exemple le brevet EP 0 611 525 B1 ou FR 2 699 370 A1) mais ne conviennent pas aux bases de lait infantiles dont la teneur en matières sèches (avant séchage) est élevée, à savoir supérieure à 15% en masse, typiquement supérieure à 20% en masse, en particulier supérieure à 25% ou à 35% en masse, essentiellement en raison d'une viscosité excessive s'opposant à la réalisation correcte d'une étape de traitement thermique, d'homogénéisation et/ou d'atomisation.

Outre les problèmes rencontrés, lors de la fabrication des laits infantiles, pour obtenir une viscosité adaptée à la consommation dudit lait par les enfants souffrant de problème de régurgitation et/ou de RGO, les fabricants sont par ailleurs confrontés à la nécessité de produire un lait satisfaisant aux normes d'hygiène réglementaires dont les critères bactériologiques sont stricts.

Si la pasteurisation des laits infantiles AR se présentant sous une forme liquide pose peu de problème, ce n'est pas le cas des laits infantiles AR se présentant sous forme de poudre. Ainsi, bien que les poudres de lait infantile présentent un faible taux d'humidité, une faible activité de l'eau (Aw), et soient conditionnées sous une très faible pression partielle en oxygène, le risque bactériologique est toujours présent et accru dès lors que des ingrédients sont ajoutés via une phase de mélange à sec sans possibilité de traitement thermique consécutif.

Bien que les problèmes de régurgitations et de reflux soient reconnus depuis longtemps, aucune composition anti-reflux et/ou anti-régurgitation entièrement satisfaisante n'a été proposée à ce jour.

Les inventeurs décrivent pour la première fois une composition anti-régurgitation et/ou anti-reflux (les expressions « anti-reflux gastro-oesophagien » et « anti-reflux » étant utilisées indifféremment dans le présent texte) sous forme de poudre, en particulier un lait infantile, ainsi que des méthodes performantes de préparation d'un tel lait infantile.

### RESUME DE L'INVENTION

La présente invention concerne ainsi une composition, en particulier une composition anti-régurgitation et/ou anti-reflux, se présentant sous une forme de poudre, en particulier un lait infantile anti-régurgitation et/ou anti-reflux, comprenant i) au moins une pectine amidée et faiblement estérifiée, et ii) au moins un épaississant et/ou un gélifiant choisi par exemple parmi la gomme xanthane, la carboxy-méthyle cellulose, l'hydroxy-propyle cellulose, l'hydroxy-méthyle cellulose, une carraghénane, un alginate, la gomme de guar et la farine de graine de caroube, de préférence choisi parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, et la gomme de guar, de manière encore plus préférée parmi la gomme xanthane, la carboxy-méthyle cellulose et un mélange de xanthane et de carboxy-méthyle cellulose. Cette composition peut avantageusement comprendre en outre iii) une pectine fortement estérifiée.

Les compositions sous forme de poudre selon l'invention présentent l'avantage considérable et inattendu d'être, une fois reconstituées, c'est-à-dire obtenues à l'aide d'un volume d'eau défini, liquide à pH7, en particulier dans un biberon, et déjà suffisamment visqueuses dans l'estomac, à un pH compris entre 6 et 3,5, en particulier entre 5,8 et 5, typiquement entre 5,5 et 5, pour diminuer de façon significative voire éliminer les symptômes de régurgitations et/ou de reflux.

Un objet particulier de l'invention concerne une boisson reconstituée, en particulier un lait infantile, à partir d'une composition, de préférence d'une composition anti-régurgitation et/ou anti-reflux, sous une forme de poudre selon l'invention.

Un autre objet particulier de l'invention concerne un concentré liquide obtenu à partir d'une telle composition sous forme de poudre selon l'invention.

La présente invention concerne également un procédé permettant l'obtention d'une composition anti-régurgitation et/ou anti-reflux sous forme de poudre, telle que décrite précédemment, en particulier d'une composition pasteurisée sous forme de poudre.

Ce procédé comprend les étapes suivantes de :
a) préparation d'une base liquide dont la teneur en matières sèches est d'au moins 20% en masse, par mélange, sous agitation, à une température d'au moins 60°C, des éléments constitutifs de ladite base,
b) homogénéisation de ladite base obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) séchage par atomisation du mélange obtenu à l'issue de l'étape b), et
d) récupération de la composition obtenu à l'issue de l'étape c) sous forme de poudre.

Le procédé précédant peut en outre comprendre une étape d'application, à la base liquide obtenue à l'issue de l'étape a) ou à l'issue de l'étape b) d'un traitement thermique, à une température comprise entre 60°C et 110°C, pendant une durée suffisante pour pasteuriser ladite base.

La composition selon l'invention est un lait infantile anti-reflux et/ou anti-régurgitation, et la base liquide est de préférence une base liquide d'aliment diététique destiné à des fins médicales spéciales, en particulier une base liquide de lait infantile.

Les éléments constitutifs de la composition comprennent au moins une pectine amidée et faiblement estérifiée, et au moins un épaississant et/ou un gélifiant choisi par exemple parmi la gomme xanthane, la carboxy-méthyle cellulose, l'hydroxy-propyle cellulose, l'hydroxy-méthyle cellulose, une carraghénane, un alginate, la gomme de guar, et la farine de graine de caroube, de préférence choisi parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, et la gomme de guar, de manière encore plus préférée parmi la gomme xanthane, la carboxy-méthyle cellulose et un mélange de xanthane et de carboxy-méthyle cellulose. Une composition particulièrement préférée comprend en outre au moins une pectine fortement estérifiée.

Un procédé particulier selon l'invention, permettant l'obtention d'un lait infantile pasteurisé, comprend les étapes suivantes de :
a) préparation d'une base liquide de lait infantile dont la teneur en matières sèches est d'au moins 20% en masse, par mélange, sous agitation, à une température d'au moins 60°C, des éléments constitutifs de ladite base, lesdits éléments constitutifs comprenant au moins une pectine amidée et faiblement estérifiée, du xanthane, et au moins une pectine fortement estérifiée,
b) homogénéisation de la base liquide de lait infantile obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) application, à la base liquide de lait infantile obtenue à l'issue de l'étape a) ou à l'issue de l'étape b) d'un traitement thermique à une température comprise entre 60°C et 110°C pendant une durée suffisante pour pasteuriser ladite base,
d) séchage par atomisation du mélange obtenu à l'issue de l'étape c), et
e) récupération du lait infantile, ledit lait infantile étant typiquement un lait infantile anti-reflux et/ou anti-régurgitation, pasteurisé obtenu à l'issue de l'étape d) sous forme de poudre.

La composition selon l'invention est avantageusement une composition susceptible d'être obtenue à l'aide d'un procédé selon la présente invention.

### DESCRIPTION DETAILLEE DE L'INVENTION

La composition selon l'invention est un lait infantile anti-reflux et/ou anti-régurgitation. Le lait infantile peut être un lait pour nourrisson (utilisable dès la naissance), un lait de suite (pour les bébés âgés de six mois au moins) ou un lait de croissance (pour les enfants âgés de un an au moins).

Classiquement les sujets concernés sont des mammifères, typiquement des êtres humains de tout âge, de préférence des nouveau-nés, des nourrissons, par exemple des bébés âgés de 6 mois ou moins, des bébés âgés de 6 à 18 mois, ou des enfants en bas âge (enfants âgés de 12 mois à trois ans).

La composition selon l'invention, typiquement la composition anti-régurgitation et/ou anti-reflux selon l'invention, se présente avantageusement sous la forme d'une poudre à reconstituer, i.e., à dissoudre dans une boisson (ou dans tout liquide consommable), typiquement de l'eau ou du lait, par exemple du lait de vache ou de chèvre, avant ingestion par le sujet présentant des symptômes de régurgitations et/ou reflux ou susceptible d'en présenter.

Un objet particulier de l'invention concerne une boisson, de préférence une boisson anti-régurgitation et/ou anti-reflux, reconstituée telle qu'une boisson à pH neutre, par exemple un lait reconstitué à partir d'une composition selon l'invention se présentant sous forme de poudre.

Typiquement, un lait infantile liquide anti-régurgitation et/ou anti-reflux selon l'invention, comprend une teneur dudit lait infantile en poudre de 11 à 15 % en masse, de préférence de 13 % en masse.

Un autre objet particulier de l'invention concerne un concentré liquide (préparé à partir d'une composition, de préférence d'une composition anti-régurgitation et/ou anti-reflux, sous forme de poudre selon l'invention) susceptible d'être dilué, de préférence à l'aide d'un liquide dont le pH est proche de la neutralité.

Typiquement, un lait anti-régurgitation et/ou anti-reflux selon l'invention se présentant sous forme de concentré liquide, préparé à partir d'une composition sous forme de poudre selon l'invention, comprend une teneur (i.e., pourcentage de matière sèche) dudit lait infantile de 25 à 50 % en masse, de préférence de 35% en masse. Un tel lait peut être dilué pour obtenir lait infantile liquide selon l'invention, typiquement un lait infantile anti-régurgitation et/ou anti-reflux, comprenant une teneur dudit lait infantile en poudre de 11 à 15 % en masse, de préférence de 13 % en masse.

La conservation du lait infantile concentré est avantageusement assurée par une stérilisation en autoclave et préférentiellement par un traitement thermique UHT suivi d'un conditionnement aseptique.

Les laits selon l'invention peuvent être administrés par voie orale ou entérale. Ils sont préférentiellement administrés par voie orale puisqu'ils ont, à côté de leur rôle alimentaire, essentiellement pour rôle de prévenir, limiter voire supprimer les symptômes de régurgitation et/ou reflux.
La composition sous forme de poudre selon l'invention présente en effet l'avantage significatif, d'être, une fois reconstituée, liquide à pH7 et visqueuse à un pH compris entre 6 et 3,5. Elle peut donc être utilisée en tant que composition anti-régurgitation et/ou anti-reflux.
Au sens de la présente invention, la viscosité du bol alimentaire est considérée comme satisfaisante si elle permet de diminuer, idéalement d'éliminer les symptômes de régurgitations et/ou de reflux.

La viscosité peut être mesurée avec un viscosimètre type Brookfield, avec un module en forme de disque ou de cylindre et à des vitesses de rotation comprises entre 20 et 100 rotations par minute. Il est également possible de mesurer le temps d'écoulement d'un volume constant de produit à travers un orifice calibré.

Avantageusement, les compositions diluées ou reconstituées selon l'invention, ont :
- à un pH compris entre 7, 3 et 6,5, une viscosité comprise entre 20 et 50 centipoises (soit entre 20 et 50 mPascal.seconde⁻¹), par exemple entre 20 et 45 centipoises, entre 20 et 30 centipoises ou entre 20 et 40 centipoises, de préférence entre 20 et 30 centipoises ;
- à un pH inférieure à 6,5, typiquement de 6 ou inférieur à 6, typiquement compris entre 5,8 et 3.5, en particulier entre 5,8 et 5 ou entre 5,5 et 3,5, de préférence entre 5,5 et 5 (par exemple 5,4, 5,3, 5,2, ou 5,1), une viscosité comprise entre 150 et 500 centipoises, par exemple entre 150 et 250, 200 et 300 ou 200 et 400, ou entre 250 et 350, de préférence entre 150 et 250 centipoises ou entre 200 et 250 centipoises.

Un objet particulier de l'invention concerne un lait infantile anti-reflux et/ou anti-régurgitation, se présentant sous la forme d'une poudre, comprenant au moins une pectine amidée et faiblement estérifiée, et au moins un épaississant et/ou un gélifiant choisi par exemple parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, une carraghénane, un alginate, la gomme de guar et la farine de graine de caroube, de préférence parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, la gomme de guar et un mélange de deux ou plusieurs, par exemple trois, desdits épaississants, de manière encore plus préférée parmi la gomme xanthane, la carboxy-méthyle cellulose et un mélange de xanthane et de carboxy-méthyle cellulose.
Des mélanges d'épaississants préférés peuvent être choisis parmi la gomme xanthane et la carboxy-methyl cellulose, la gomme xanthane et l'hydroxy-propyle cellulose, la gomme xanthane et la gomme de guar, la gomme xanthane et l'hydroxy-propyle méthyle cellulose, la gomme xanthane et la méthyle cellulose, la gomme xanthane, la gomme de guar et la carboxy-methyl cellulose, la carboxy-méthyle cellulose et la gomme de guar, la carboxy-methyle cellulose et l'hydroxy-propyle méthyle cellulose, la carboxy méthyle cellulose et la méthyle cellulose, l'hydroxy-méthyle cellulose et la méthyle cellulose, la méthyle cellulose, la gomme de guar et la l'hydroxy-méthyle cellulose, de préférence parmi la gomme xanthane et la carboxy-methyl cellulose, la gomme xanthane et la gomme de guar, la gomme xanthane et l'hydroxy-propyle cellulose, la gomme xanthane, la gomme de guar et la carboxy-methyl cellulose.

Un lait reconstitué à partir d'un lait sous forme de poudre selon l'invention, typiquement d'un lait anti-régurgitation et/ou anti-reflux sous forme de poudre selon l'invention, présente une viscosité particulièrement adaptée dès pH 6, et plus généralement à un pH compris entre 6 et 5, par exemple entre 5,8 et 5,5, typiquement à un pH de 5,2, 5,3, 5,4, 5,5, 5,6 et 5,7.
Il est donc facile à boire par la tétine du biberon et permet au sujet de s'alimenter sans frustration tout en limitant les risques d'aérophagie. Il développe par ailleurs plus rapidement que les laits anti-régurgitation connus (quelques minutes (typiquement entre 5 et 10 minutes), de préférence 5 minutes, contre environ 30 à 60 minutes) la viscosité souhaitée dans l'estomac pour limiter voire éliminer les symptômes de régurgitation et/ou de reflux (RGO). Le pH requis pour le développement d'une viscosité satisfaisante au sens de l'invention est donc significativement supérieur pour la composition selon l'invention au pH requis, d'environ 3,5, pour les compositions AR connues.

La viscosité optimale souhaitée du lait infantile anti-régurgitation et/ou anti-reflux selon l'invention est de préférence inférieure à 50 mPa.s mesurée à un pH proche de la neutralité et à une température comprise entre 35°C et 40°C, et supérieure à 160 mPa.s, de préférence à 200 mPa.s, encore plus préférentiellement comprise entre 200 et 300 mPa.s, lorsqu'elle est mesurée à pH= 5,5 et à une température également comprise entre 35°C et 40°C.

Les éléments constitutifs des compositions selon l'invention constituent le mélange identifié dans le cadre de la présente invention par le terme « base ».
Typiquement, les éléments constitutifs des compositions diététique ou nutritionnelle selon l'invention constituent le mélange identifié dans le cadre de la présente invention par le terme « base de composition diététique ou nutritionnelle » et les éléments constitutifs des laits infantiles constituent le mélange identifié dans le cadre de la présente invention par l'expression « base de lait infantile ».

Un lait infantile anti-régurgitation et/ou anti-reflux selon la présente invention peut comprendre toute base de lait infantile connue de l'homme du métier. Ainsi, n'importe quelle base de lait infantile, dont les propriétés nutritionnelles sont adaptées au besoin des nourrissons et enfants, y compris les Aliments Diététiques Destinés à des Fins Médicales Spéciales (ADDFMS), peut être utilisée pour préparer un lait infantile selon la présente l'invention.

Une base de lait infantile standard comprend des glucides, des lipides, des protéines, des minéraux, des vitamines et éventuellement des facteurs de croissance. Les proportions habituelles de ces différents constituants au sein de la base de lait sont d'environ 55% pour les glucides, 25% pour les lipides, 15% pour les protéines et 5% pour l'ensemble constitué par les minéraux et vitamines, les pourcentages étant calculés par rapport à la masse totale de matière sèche de la base de lait déshydratée.

La base de lait peut en outre éventuellement comprendre d'autres composés connus de l'homme du métier tels que les composés améliorant la texture du lait, le goût du lait et/ou présentant un intérêt nutritionnel ou fonctionnel spécifique (nucléotides, probiotiques, prébiotiques, etc.).

Classiquement, la fraction protéique de la base de lait infantile comprend deux types de protéines : des protéines d'origine animale, notamment celles issues du lait (caséine et protéines solubles, encore appelées protéines du lactosérum), et des protéines d'origine végétale. La fraction protéique peut toutefois comprendre l'un seulement de ces deux types de protéines.

Les protéines d'origine animale peuvent provenir par exemple de lait de vache, de lait de chèvre, de lait humain, de lait de chamelle, de lait de bufflonne, lait d'ânesse et/ou de lait de jument.
Les protéines d'origine végétale peuvent provenir par exemple du riz, du soja et/ou du pois.

Les protéines présentes dans la base de lait infantile, utilisée dans le cadre de la présente invention, peuvent être entières ou, au contraire, totalement ou partiellement hydrolysées. Les protéines hydrolysées ont de préférence un degré d'hydrolyse compris entre environ 5% et environ 90%, de préférence entre environ 5% et environ 50%. Le degré d'hydrolyse correspond au nombre de liaisons peptidiques rompues par l'hydrolyse. Plus ce nombre est élevé, plus l'hydrolyse est poussée.

Dans un mode de réalisation particulier au sens de la présente invention, le degré d'hydrolyse des protéines hydrolysées est compris entre environ 20% et environ 50%, de préférence entre environ 20% et environ 40%.

La fraction protéique peut également comprendre des acides aminés en mélange. Les acides aminés en mélange peuvent être des acides aminés naturels, des acides aminés de synthèse ou un mélange d'acides aminés naturels et d'acides aminés de synthèse.

Les acides aminés en mélange peuvent constituer à eux seuls la fraction protéique du lait infantile anti-régurgitation selon la présente invention. Ils peuvent également être présents à côté des protéines hydrolysées et des éventuelles protéines non hydrolysées.

Ces protéines hydrolysées et/ou acides aminés en mélange sont plus digestes que les protéines non hydrolysées et permettent d'accélérer la vidange gastrique. Une composition préférée selon l'invention, en particulier une composition anti-régurgitation et/ou anti-reflux préférée, comprend des protéines hydrolysées et/ou des acides aminés en mélange.

Une composition particulière selon l'invention, typiquement une composition anti-régurgitation et/ou anti-reflux particulière, comprend une fraction protéique contenant une majorité de protéines hydrolysées et/ou d'acides aminés en mélange.

Une autre composition particulière selon l'invention, typiquement une autre composition anti-régurgitation et/ou anti-reflux particulière, comprend une fraction protéique contenant une majorité de protéines non hydrolysées.

Les lipides typiquement susceptibles de rentrer dans la composition du lait infantile selon la présente invention peuvent être choisis par exemple parmi la matière grasse du lait, l'huile de carthame, les lipides de jaune d'oeuf, l'huile d'olive, l'huile de noix de coco, l'huile de palme, l'huile de soja, l'huile de tournesol, l'huile de poisson, les huiles issues d'algues et/ou de champignons, l'oléine de palme, les triglycérides à chaînes moyennes, et les esters d'acides gras choisis, par exemple, parmi l'acide arachidonique, l'acide linoléique, l'acide palmitique, l'acide stéarique, l'acide docosahexanoïque, l'acide eicosapentanoique, l'acide linolénique, l'acide oléique, l'acide laurique, l'acide caprique, l'acide caprylique et l'acide caproïque.

Les glucides susceptibles de rentrer dans la composition du présent lait infantile (autre que les agents épaississants décrits plus tard dans la présente description) peuvent être n'importe quel sucre connu de l'homme de l'art comme étant adapté à la nutrition humaine, typiquement infantile. Typiquement, les glucides peuvent être choisis parmi le lactose, les maltodextrines ou sirop de glucose, le saccharose, le fructose, et le glucose.

Des exemples de sels minéraux, de vitamines et d'autres nutriments éventuellement présents dans le lait infantile anti-régurgitation selon l'invention incluent la vitamine A, la vitamine B6, la vitamine B12, la vitamine D, en particulier la vitamine D3 (cholécalciférol), la vitamine E, la vitamine K, la vitamine C, l'acide folique, la thiamine, l'inositol, la riboflavine, la niacine, la biotine, l'acide pantothénique, la choline, le calcium, le phosphore, l'iode, le fer, le magnésium, le cuivre, le zinc, le manganèse, le chlore, le potassium, le sodium, le sélénium, le chrome, le molybdène, la taurine, et la L-Carnitine.

En plus des considérations de compatibilité et de stabilité liées aux procédés de préparation décrits dans le cadre de la présente invention et aux conditions de stockage d'un lait selon l'invention, la présence et les quantités de sels minéraux et de vitamines spécifiques éventuellement présents pourront changer légèrement selon la population ciblée (nourrissons ou enfants âgés de 6 à 18 mois par exemple).

Dans le contexte de la présente invention, les termes « pectine » et « substance pectique » sont indifféremment employés. Les substances pectiques sont des polymères de polysaccharides acides. Elles sont composées d'une chaîne principale constituée de monomères d'acide uronique liés en 1-4 entre lesquels s'intercalent des molécules de rhamnoses par des liaisons 1-2 et 1-4 responsables de la forme de zigzag des macromolécules de pectine. Ces molécules complexes présentent des ramifications au niveau des acides uroniques comme au niveau du rhamnose par des molécules de type galactanes, rhamnanes, etc.

Il existe une grande variété de pectines dont l'origine est exclusivement végétale. Les pectines sont présentes en grande quantité dans les pépins et les zestes de groseilles, pommes, coings et agrumes.

Les pectines « faiblement estérifiées » utilisées dans le cadre de la présente invention sont typiquement des pectines d'agrumes.

Le degré d'amidation de la « pectine amidée et faiblement estérifiée », présente dans la composition selon l'invention, est compris entre environ 5% et environ 30%, de préférence entre environ 5% et environ 20%, plus préférentiellement entre environ 10% et environ 20%.
Son degré d'estérification, typiquement de méthylation, est typiquement compris entre environ 20% et environ 50%, préférentiellement entre environ 30% et environ 50%, et plus préférentiellement entre environ 30% et environ 40%.

Lorsque la composition selon l'invention est un lait infantile, la présence, au sein de cette composition, d'au moins une pectine amidée faiblement estérifiée, permet d'obtenir un lait infantile reconstitué (après la dissolution de la poudre dans l'eau) présentant une faible viscosité, typiquement un lait liquide présentant une viscosité comprise entre 20 et 50 centipoises à un pH proche de la neutralité.

Dans les compositions selon l'invention, typiquement dans les laits infantiles anti-régurgitation et/ou anti-reflux, la « pectine faiblement estérifiée » est présente à une concentration comprise entre environ 1% et environ 10%, de préférence entre environ 2% et environ 8%, par exemple entre environ 3% et environ 8%, de manière encore plus préférée entre environ 3% et environ 6%, et est typiquement de 4%.

Avantageusement, la concentration, au sein d'une composition selon l'invention, de pectine faiblement estérifiée et éventuellement de pectine fortement estérifiée sera adaptée à la nature et à la quantité des protéines éventuellement présentes dans la composition.

Avantageusement, la concentration, au sein d'une composition selon l'invention, de pectine faiblement estérifiée, en particulier de pectine amidée et faiblement estérifiée, sera d'autant plus élevée que ladite composition sera riche en protéines hydrolysées et, inversement, cette concentration sera d'autant plus basse que ladite composition sera riche en protéines non hydrolysées.

Dans une composition particulière selon l'invention la concentration de protéines non hydrolysées est comprise entre environ 13 et 14% et celle de pectine faiblement estérifiée est d'environ 3%.

Dans une autre composition particulière selon l'invention la concentration d'acides aminés en mélange est comprise entre environ 13 et 14% et celle de pectine faiblement estérifiée est d'environ 4%.

Les pectines « hautement estérifiées » ou « fortement estérifiées » utilisables dans le cadre de la présente invention sont typiquement des pectines d'agrumes, en particulier des pectines d'agrumes non amidées.
Les inventeurs ont découvert qu'une telle « pectine fortement estérifiée », utilisée en combinaison avec au moins une « pectine faiblement estérifiée », de préférence avec au moins une « pectine amidée et faiblement estérifiée », permet d'améliorer la stabilité de la composition anti-régurgitation et/ou anti-reflux selon l'invention (en particulier la stabilité des protéines) à pH acide, i.e., à un pH inférieur à 4.

Le degré d'estérification, typiquement de méthylation, de la « pectine fortement estérifiée » présente dans la composition selon l'invention est compris entre environ 50% et environ 90%, de préférence entre environ 50% et environ 80%, plus préférentiellement entre environ 60% et environ 70%.

L'indice de stabilité de ladite pectine à pH = 4 est compris entre 140-200, de préférence entre environ 150 et environ 190, plus préférentiellement entre environ 165 et environ 185. Cet indice est une mesure par sédimentation (comprise entre 100 et 200) de la capacité de la pectine hautement estérifiée à protéger les protéines non-hydrolysées en milieu acide.

Dans les compositions selon l'invention, typiquement dans les laits infantiles anti-régurgitation et/ou anti-reflux, la « pectine fortement estérifiée » est avantageusement présente à une concentration comprise entre environ 0.1% et environ 10%, de préférence entre environ 0.1% et environ 8%, plus préférentiellement entre environ 1% et environ 3%.

De manière préférée, la concentration de pectine hautement estérifiée, au sein d'une composition selon l'invention comprenant au moins une pectine faiblement estérifiée, de préférence moins une pectine amidée et faiblement estérifiée, et au moins une pectine fortement estérifiée, sera d'autant plus élevée que ladite composition sera riche en protéines non hydrolysées et, inversement, cette concentration sera d'autant plus basse que ladite composition sera riche en protéines hydrolysées et/ou acides aminés en mélange.

Dans une composition particulière selon l'invention la concentration de protéines non hydrolysées est comprise entre environ 13 et 14%, celle de pectine faiblement estérifiée, typiquement celle de pectine amidée et faiblement estérifiée, est d'environ 3%, et celle de pectine fortement estérifiée est comprise entre environ 3 et 4,5% (elle est typiquement de 4%).

Dans une composition particulière selon l'invention la concentration de protéines fortement hydrolysées est comprise entre environ 13 et 14%, celle de pectine faiblement estérifiée, typiquement celle de pectine amidée et faiblement estérifiée, est d'environ 4%, et celle de pectine fortement estérifiée est nulle ou d'environ 1%.

Comme indiqué précédemment, la composition selon l'invention, typiquement la composition anti-régurgitation et/ou anti-reflux, comprend au moins une pectine faiblement estérifiée, de préférence au moins une pectine amidée et faiblement estérifiée, et au moins un épaississant et/ou un gélifiant choisi par exemple parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, une carraghénane, un alginate, la gomme de guar et la farine de graine de caroube, de préférence parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, la gomme de guar et un mélange de deux ou plusieurs, par exemple trois, desdits épaississants (tels que ceux identifiés dans le présent texte), de manière encore plus préférée parmi la gomme xanthane et la carboxy-méthyle cellulose.

Un objet particulier de l'invention est un lait anti-régurgitation et/ou anti-reflux selon l'invention, comprenant, pour 100 grammes de matière sèche, 0,5 à 10 grammes, de préférence entre 3 et 8 grammes, de pectines (« pectine faiblement estérifiée » et/ou « pectine fortement estérifiée »), et 0,5 à 8 grammes, de préférence entre 0,5 et 4 grammes, typiquement entre 0,5 et 1,5 grammes, du ou des épaississants et/ou gélifiants choisis.

Parmi les épaississants d'origine végétale présents dans les compositions selon l'invention à côté de la ou des pectines, des épaississants et/ou gélifiants préférés sont avantageusement choisis parmi la gomme xanthane et la carboxy-méthyle cellulose. Un épaississant dont l'usage est particulièrement préféré est la gomme xanthane encore identifié, dans le cadre de la présente invention, sous le terme « xanthane ».

Le xanthane est un polyoside ramifié, classiquement utilisée comme additif alimentaire sous le code E415, obtenu à partir de l'action d'une bactérie, la *Xanthomonas campestris.* Il est constitué d'une combinaison de quatre sous-unités : le glucose, le mannose, l'acide glucuronique et l'acide pyruvique.

Les inventeurs ont découvert que le xanthane est un épaississant parfaitement adapté à une utilisation, dans une composition selon l'invention, en particulier dans un lait infantile, en combinaison avec une ou deux des pectines précédemment mentionnées (« faiblement estérifiée » d'une part et « fortement estérifiée » d'autre part). Les inventeurs ont notamment démontré qu'une telle combinaison permettait d'obtenir une composition d'aspect lisse et non gélifié en milieu aqueux à un pH proche de la neutralité, en particulier au pH de reconstitution du biberon de lait, et présentant une viscosité homogène, en particulier à un pH de 6 ou inférieure à 6, de préférence compris entre 5,5 et 3,5, permettant de limiter, idéalement d'éliminer les symptômes de régurgitation et/ou de reflux grâce à l'obtention rapide de la viscosité souhaitée.
Les inventeurs ont en outre constaté l'effet stabilisant, en milieu acide tout comme en milieu neutre, exercé par le xanthane utilisé en combinaison avec une ou deux des pectines précédemment mentionnées, sur la composition selon l'invention.

Les compositions selon l'invention peuvent en outre comprendre de l'amidon, en particulier de l'amidon pré-cuit ou prégélatinisé, de préférence de l'amidon prégélatinisé.

L'amidon est un mélange de 2 homopolymères, l'amylose et l'amylopectine composés d'unités D-Anhydroglucopyranose (AGU) qui appartiennent à la famille des polysaccharides. Les unités AGU sont liées entre elles par des liaisons α (1-4), en général caractéristique des polyosides de réserve et des liaisons α (1-6) qui sont à l'origine de ramifications dans la structure de la molécule. Ces 2 homopolymères, qui diffèrent par leur degré de branchement et leur degré de polymérisation sont:
- L'amylose, légèrement ramifié avec de courtes branches et dont la masse moléculaire peut être comprise entre 10 000 et 1 000 000 Daltons. La molécule est formée de 600 à 1000 molécules de glucose.
- l'amylopectine ou isoamylose, molécule ramifiée avec de longues branches toutes les 24 à 30 unités glucoses par l'intermédiaire des liaisons α (1-6).
La masse moléculaire de l'amidon est comprise entre 1 000 000 et 100 000 000 Daltons, et son niveau de branchement est de l'ordre de 5%. La chaîne totale peut faire entre 10 000 et 100 000 unités glucoses.
Le ratio entre l'amylose et l'amylopectine dépend de la source botanique de l'amidon. L'amidon prégélatinisé est obtenu en chauffant une suspension dans l'eau (lait d'amidon). Cette suspension est instable mais chauffée devient visqueuse et translucide.

Un lait particulier, typiquement un lait infantile anti-régurgitation et/ou anti-reflux selon la présente invention, dont la base protéique est constituée par exemple d'un hydrolysat poussé de protéines animales ou végétales (degré d'hydrolyse compris entre 20 et 30%), peut avantageusement comprendre :
- entre 1% environ et 10% environ, de préférence entre 1% environ et 5 % environ, encore plus préférentiellement entre 2% environ et 4% environ en pourcentage de masse de matière sèche de pectine faiblement estérifiée,
- entre 0,1% environ et 10% environ, de préférence entre 0,1% environ et 8% environ, encore plus préférentiellement d'environ 1% de masse de matière sèche de pectine fortement estérifiée, et
- entre 0,1% environ et 2% environ, de préférence entre 0,1% environ et 1% environ, encore plus préférentiellement entre 0,5% et 0,9% environ de masse de matière sèche de xanthane par rapport à la masse de matière sèche totale de lait.

Un tel lait peut en outre comprendre entre 0,1% environ et 10% environ, de préférence entre 0,1 % environ et 5% environ, encore plus préférentiellement entre 0,1 % environ et 2% en pourcentage de masse de matière sèche d'amidon prégélatinisé.

De telles proportions sont favorables à l'augmentation de la viscosité du lait dans les conditions précédemment décrites.

La base de lait infantile utilisée dans le cadre de la présente invention se présente classiquement sous forme liquide et comprend typiquement une teneur en matière sèche (extrait sec) d'environ 25% à 60% en masse, de préférence d'environ 30% à 50%, d'environ 30% à 40% en masse, ou d'environ 25% à 45% en masse, encore plus préférentiellement d'environ 30% à 40% en masse ou d'environ 35% à 40% en masse.

Les pectines et épaississants et/ou gélifiants utilisées dans le cadre de la présente invention peuvent se présenter sous forme de poudre ou sous forme de solution aqueuse. Au sens de l'invention, les expressions « milieux aqueux » ou « solution aqueuse » désignent respectivement un milieu ou une solution qui est au moins partiellement constituée d'eau.

Un lait anti-régurgitation et/ou anti-reflux particulier selon l'invention se présentant sous forme de poudre comprend avantageusement au moins 94% de matière sèche, de préférence au moins 95% de matière sèche, de manière encore plus préférée au moins 98% de matière sèche.

La présente invention concerne également un procédé permettant l'obtention d'une composition sous forme de poudre, en particulier d'une composition diététique ou nutritionnelle telle que décrite précédemment, de préférence d'un lait infantile, de manière encore plus préférée d'un lait infantile anti-régurgitation et/ou anti-reflux, tel que décrit précédemment.

Ce procédé comprend les étapes suivantes de :
a) préparation d'une base liquide dont la teneur en matières sèches est d'au moins 20% en masse, par mélange, sous agitation, à une température d'au moins 60°C, des éléments constitutifs de ladite base,
b) homogénéisation de ladite base obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) séchage par atomisation du mélange obtenu à l'issue de l'étape b), et
d) récupération de la composition obtenu à l'issue de l'étape c) sous forme de poudre.

Le procédé précédant permet, contrairement aux procédés connus à ce jour, de préparer, de façon simple et efficace, à partir des éléments constitutifs d'une base, typiquement à partir d'une base de lait infantile se présentant sous forme liquide, une composition selon l'invention, de préférence une composition anti-régurgitation et/ou anti-reflux, typiquement un lait infantile anti-régurgitation et/ou anti-reflux, qui va être séchée et transformée en poudre. La poudre obtenue à l'issue de ce procédé est homogène, contrairement aux poudres obtenues par mélange à sec décrites précédemment, et la composition liquide reconstituée à partir d'une telle poudre présente les propriétés de viscosité, décrites précédemment, requises pour limiter, idéalement supprimer, les symptômes de régurgitation et/ou de reflux.

La composition sous forme de poudre selon l'invention peut en outre avantageusement être une composition pasteurisée.

Au sens de la présente invention, la pasteurisation désigne une étape de traitement thermique provoquant la destruction des germes considérés comme pathogènes pour le sujet auquel la composition est destinée, et plus généralement une diminution de la flore bactérienne.
Classiquement le traitement thermique est réalisé à une température comprise entre environ 60°C et environ 110°C pendant une durée comprise entre environ 15 minutes et quelques secondes, par exemple environ 25 ou 30 secondes. L'homme du métier est en mesure de déterminer la durée adaptée à une température donnée qui va permettre d'obtenir la pasteurisation souhaitée sans détruire la composition, ses propriétés nutritives ou ses propriétés anti-régurgitation et/ou reflux. Concernant le lait en particulier, L'homme du métier est en mesure de déterminer les conditions adaptées à la préservation des protéines et des vitamines.

Pour le lait, en particulier le lait infantile, la flore totale maximale tolérée ne doit pas excéder 1000 CFU (colonies formant unité) par gramme de poudre.
Parmi cette flore, *Clostridium perfringens, Escherichia coli, Bacillus cereus, Listeria monocytogenes,* les Staphylocoques à coagulase +, les salmonelles et les entérobactéries (notamment *Cronobacter sakazaki)* sont en particulier considérées comme pathogènes et doivent de préférence être totalement éliminés. Le procédé selon l'invention, décrit précédemment, permet d'éliminer l'ensemble des bactéries pathogènes (en particulier les bactéries listées précédemment) pour l'homme, en particulier l'enfant.

Le procédé décrit précédemment peut en outre comprendre de façon avantageuse une étape additionnelle d'application, à la base liquide obtenue à l'issue de l'étape a) ou à l'issue de l'étape b) d'un traitement thermique à une température comprise entre 60°C et 110°C pendant une durée suffisante pour pasteuriser la base liquide. Ce procédé permet ainsi l'obtention d'une composition, typiquement d'une composition anti-régurgitation et/ou anti-reflux, pasteurisée sous forme de poudre.
Une telle composition ne présente pas les risques microbiologiques (notamment pour *C. Sakazakii)* observés lors des mélanges à sec d'une base de lait infantile avec de la poudre d'amidon ou de caroube.

Un objet particulier de la présente invention concerne ainsi un procédé permettant l'obtention d'une composition, typiquement d'une composition anti-régurgitation et/ou anti-reflux, pasteurisée sous forme de poudre, en particulier un lait infantile ou une composition diététique tels que décrit précédemment, comprenant les étapes suivantes de:
a) préparation d'une base liquide dont la teneur en matières sèches est d'au moins 20% en masse, par mélange, sous agitation, à une température d'au moins 60°C, des éléments constitutifs de ladite base de lait infantile,
b) homogénéisation de la base liquide obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) application, à la base liquide obtenue à l'issue de l'étape a) ou à l'issue de l'étape b) d'un traitement thermique à une température comprise entre 60°C et 110°C pendant une durée suffisante pour pasteuriser ladite base,
d) séchage par atomisation du mélange obtenu à l'issue de l'étape c), et
e) récupération de la composition pasteurisée obtenu à l'issue de l'étape d) sous forme de poudre.

L'étape a) de préparation d'une base liquide comprend le mélange, sous agitation, des composants, ingrédients ou éléments d'intérêt, tels que décrits précédemment, constitutifs de ladite base.

La réalisation d'un mélange liquide passe par la dilution de chaque ingrédient dans de l'eau.
Les ingrédients (base de la composition, par exemple base de lait infantile ou base de composition diététique, et agent(s) épaississant(s)) peuvent être mélangés sous forme de poudres et mis en solution par la suite. Ils peuvent aussi être mélangés sous forme de solutions. Il est également envisageable d'ajouter l'un des composants sous forme de poudre à l'autre composant qui se trouve en solution. Il est dans ce cas préférable de maintenir le composant en solution sous agitation lors du mélange au composant sous forme de poudre afin de limiter, idéalement d'éviter la formation d'agglomérats lors du mélange.
Ainsi, le caractère aqueux du mélange obtenu à l'issue de l'étape a) peut provenir de la forme liquide des agents épaississants, de la forme liquide de la base utilisée et/ou de l'adjonction d'eau au mélange des produits utilisés sous forme de poudre.

Un agitateur ou une unité de mélange, par exemple une pompe de mélange, un défloculateur, ou un mélangeur équipé d'un système rotor/stator, peut avantageusement être utilisé pour dissoudre les différents ingrédients et faciliter l'obtention d'une base homogène.

L'utilisation préférée d'un mélangeur de forme et taille adaptées permet également d'éviter l'incorporation excessive d'air dans la base liquide, en particulier la base de lait.
L'homme du métier sera par ailleurs en mesure d'adapter les vitesses de rotation pour diminuer encore davantage une telle incorporation excessive d'air dans la base liquide. L'étape a) de mélange est de préférence réalisée à une température d'au moins 60°C, par exemple comprise entre environ 60°C et 90°C ou entre environ 60 °C et 80 °C, encore plus préférentiellement entre environ 70 °C et 75°C. Typiquement, la température est de 75°C.

Dans un mode de réalisation préféré, la base liquide obtenue à l'issue de l'étape a) est maintenue sous agitation jusqu'à l'application de l'étape d'homogénéisation, par exemple à l'aide d'un dispositif tel que décrit précédemment.

L'étape d'homogénéisation de la base liquide mélangée, éventuellement pasteurisée, obtenu à l'issue de l'étape a) du procédé selon l'invention permet le fractionnement des éléments constitutifs de ladite base. L'homogénéisation comprend deux étapes de compression destinée à renforcer la stabilité de cette base. La première étape i) de fractionnement est de préférence réalisée sous une pression comprise entre environ 100 bars et environ 300 bars, la deuxième étape ii) étant de préférence réalisée sous une pression comprise entre 10 bars environ et 60 bars environ.
Cette étape est avantageusement mise en oeuvre sur un homogénéisateur à deux étages.
La pression d'homogénéisation du premier étage dudit homogénéisateur est ainsi typiquement comprise entre environ 100 bars et environ 300 bars, de préférence entre environ 150 bars et environ 300 bars, encore plus préférentiellement entre environ 170 bars et environ 200 bars.
La pression d'homogénéisation du second étage est typiquement comprise entre environ 10 bars et environ 60 bars, de préférence entre environ 30 bars et environ 60 bars, encore plus préférentiellement entre environ 30 bars et environ 40 bars.

L'étape de pasteurisation, éventuellement présente dans le procédé de préparation selon l'invention, prévoit l'application, au mélange obtenu à l'issu de l'étape a) ou à l'issue de l'étape d'homogénéisation, d'un traitement thermique compris entre environ 70°C et environ 110°C, de préférence entre 70°C et 100°C, entre 75°C et 100°C, entre 75°C et 95°C, entre 80°C et 95°C ou entre 85°C et 95°C, encore plus préférentiellement entre 80°C et 90°C, pendant une durée suffisante pour inactiver et détruire au moins les germes considérés comme pathogènes (notamment C. *Sakazakü).*

Typiquement, le traitement thermique de l'étape de pasteurisation est appliqué pendant au moins 2 minutes, et de préférence au plus 10 minutes, lorsque la température est égale ou inférieure à 80°C, par exemple comprise entre 75°C et 60°C, et pendant au moins 25 secondes, typiquement au moins 1 minute, et de préférence au plus 5 minutes, lorsque la température est égale ou supérieure à 85°C, par exemple comprise entre 85°C et 100°C.

Le traitement peut également être appliqué pendant une durée supérieure à 2 minutes, et de préférence inférieure à 10 minutes, lorsque la température est de 75°C, pendant une durée comprise entre environ 2 minutes et environ 3 minutes lorsque la température est de 80°C, pendant une durée comprise entre environ 1 minute et environ 2 minutes lorsque la température est de 90°C, pendant une durée d'environ 1 minute lorsque la température est de 95°C, et de moins de 30 secondes, typiquement de 25 secondes, lorsque la température est de 100°C.

Le mélange, pasteurisé ou non, obtenu à l'issue de l'étape d'homogénéisation du procédé selon l'invention est avantageusement séché par atomisation afin d'obtenir une composition (de préférence une composition anti-régurgitation et/ou anti-reflux) sous forme de poudre comprenant, comme expliqué précédemment, un extrait sec compris entre 85% et 99%, de préférence un extrait sec d'au moins 94% ou d'au moins 95%, encore plus préférentiellement d'au moins 98%.

Le mélange, pasteurisé ou non, obtenu à l'issue de l'étape d'homogénéisation du procédé selon l'invention est typiquement introduit au sommet d'une tour d'atomisation. Le mélange est alors "atomisé" (transformé en aérosol ou brouillard) au moyen d'une turbine d'atomisation ou par injection à haute pression au travers d'une ou plusieurs buses. Les gouttelettes ainsi formées sont entraînées et déshydratées par un courant d'air chaud dont la température est typiquement comprise entre 160°C et 240°C, de préférence entre 180°C et 220°C. Les gouttelettes sont séchées en une poudre avant de tomber sur les parois inférieures de l'appareil. La séparation poudre - air humide est obtenue par exemple à l'aide de séparateurs cyclone dont l'utilisation est bien connue de l'homme de l'art.

Lorsque l'on souhaite obtenir une composition en poudre selon un tel procédé, la déshydratation dans la tour d'atomisation doit de préférence ne pas être totale. L'humidité résiduelle présente dans la composition en poudre peut par exemple être comprise entre 6 et 14% en bas de chambre. Cette humidité résiduelle permet une agglomération limitée et contrôlée des particules qui conduit à la formation de granulés à structure poreuse.

La déshydratation peut ensuite être achevée dans des dispositifs complémentaires de type sécheurs à lit fluidisé. La poudre peut ensuite être refroidie à l'intérieur d'un lit vibro-fluidisé.

Il également possible de préparer une composition anti-régurgitation et/ou anti-reflux en poudre selon l'invention (typiquement une composition anti-régurgitation et/ou anti-reflux permettant par exemple la reconstitution d'un lait présentant les propriétés décrites précédemment, par exemple un lait liquide à un pH d'environ 7 et visqueux à un pH compris entre environ 6 et environ 3,5, en particulier à un pH compris entre 5,8 et 5) par mélange à sec d'une base (recueillie sous forme de poudre en sortie de tour de séchage) avec un ingrédient d'intérêt tel que décrit précédemment, typiquement au moins une pectine amidée faiblement estérifiée, et au moins un épaississant et/ou un gélifiant choisi par exemple parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, une carraghénane, un alginate, la gomme de guar et la farine de graine de caroube, de préférence parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, la gomme de guar et un mélange de deux ou plusieurs, par exemple trois, desdits épaississants (tels que ceux identifiés dans le présent texte), de manière encore plus préférée parmi la gomme xanthane et la carboxy-méthyle cellulose.

D'autres avantages et applications de la présente invention apparaîtront à la lecture des exemples qui suivent, qui doivent être considérés comme purement illustratifs et non limitatifs.

### LEGENDES DES FIGURES

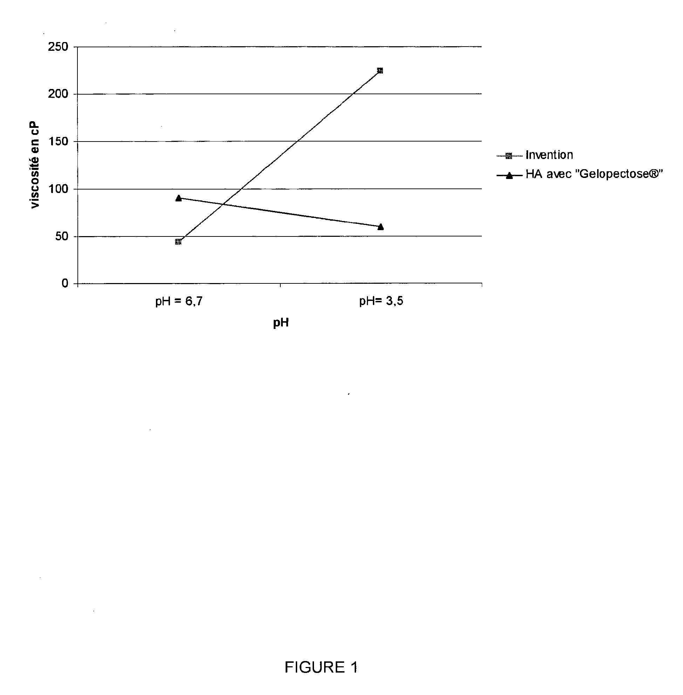

La Figure 1 représente l'évolution de la viscosité des produits (mesurée en centipoises cP) selon le pH d'un lait infantile réalisé selon la présente invention et d'un lait Hypoallergénique (HA) reconstitué avec ajout de Gélopectose. L'abscisse du graphique présente donc les valeurs de pH : 6,7 et 3,5 et l'ordonnée les valeurs de viscosités en centipoises.

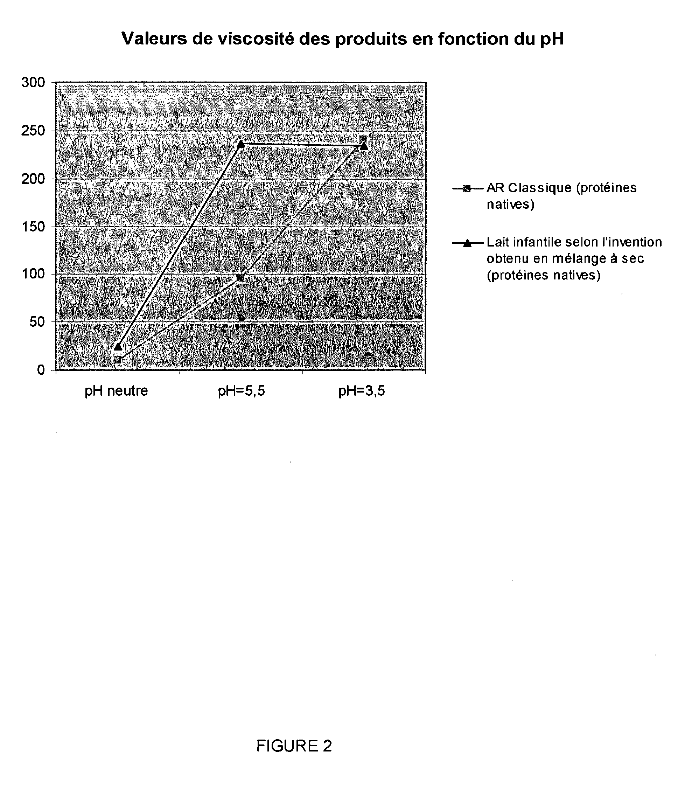

La Figure 2 représente l'évolution de la viscosité des produits (mesurée en centipoises cP) selon le pH du lait infantile reconstitué à 13% d'extrait sec. L'abscisse du graphique présente donc les valeurs de pH : neutre ; 5,5 et 3,5 et l'ordonnée les valeurs de viscosité en centipoises.

### EXEMPLES :

### EXEMPLE 1 : Formulation

La formulation des laits destinés à l'alimentation infantile est le plus souvent strictement encadrée par une législation fixant des normes de composition. Selon les pays, il peut y avoir des différences d'appréciation en raison notamment de spécificités locales dans la pratique de la diversification alimentaire ou bien des modifications mineures des optimums nutritionnels résultants de travaux ou d'études conduites localement.
Dans ces conditions, le présent exemple n'entend pas représenter la diversité des formulations de laits épaissies selon l'invention.

Classiquement, pour répondre aux besoins nutritionnels du nourrisson, les laits infantiles comprennent environ 10-15% de protéines, environ 25% de lipides et environ 50 à 65% de glucides ainsi que des minéraux, des vitamines et éventuellement des facteurs de croissance. D'autres ingrédients tels que par exemple un ou plusieurs prébiotiques et/ou probiotiques peuvent par ailleurs être ajoutés aux laits infantiles.

Un exemple d'une composition de lait infantile anti-régurgitation et/ou anti-reflux selon l'invention est fourni dans le tableau I ci-dessous.

**Tableau I :**

| | | Pour 100 g de poudre | Pour 100 ml de lait reconstitué, à 13% |
|---|---|---|---|
| Protéines | g | 12,1 | 1,6 |
| Lipides dont: | g | 26,2 | 3,41 |
| - Acide Linoléique | g | 4,5 | 0,6 |
| - Acide a-linolénique | mg | 450 | 58,5 |
| Glucides dont: | g | 52,7 | 6,85 |
| - Maltodextrines | g | 46,85 | 4,0 |
| - Lactose | g | 0 | 0 |
| - Amidon | g | 0,94 | 0,12 |
| Pectine HM | g | 1,0 | 0,4 |
| Pectine amidée LM | g | 4,0 | 0,52 |
| Xanthane | g | 0,7 | 0,09 |
| Energie | kcal | 495,9 | 64,5 |
| Minéraux dont : | g | 4,4 | 0,57 |
| - Sodium | mg | 230 | 29,9 |
| - Potassium | mg | 610 | 79,3 |
| - Chlore | mg | 340 | 44,2 |
| - Calcium | mg | 620 | 80,6 |
| - Phosphore | mg | 340 | 44,2 |
| - Magnésium | mg | 50 | 6 |
| - Fer | mg | 6 | 0,8 |
| - Zinc | mg | 4 | 0,52 |
| - Iode | µg | 70 | 9,10 |
| - Cuivre | µg | 350 | 45,5 |
| - Manganèse | µg | 50 | 6,5 |
| - Sélénium | µg | 10 | 1,3 |
| - Molybdène | µg | <45 | <5,8 |
| - Chrome | µg | <45 | <5,8 |
| - Fluor | µg | <980 | <127 |
| Mélange de vitamines | g | 0,3 | 0,039 |

### EXEMPLE 2 : Procédé de fabrication d'un lait infantile pasteurisé sous forme de poudre comprenant i) une pectine amidée et faiblement estérifiée et ii) du xanthane :

La base de lait infantile comprenant 40% d'extrait sec est préparée en mélangeant de l'eau préalablement chauffée à 75°C avec les différents ingrédients du lait infantile (protéines, pectines amidées et faiblement estérifiées, glucides, xanthane, minéraux matières grasses végétales, vitamines et facteurs de croissance). Les pectines sont incorporées dans la base de lait infantile maintenue sous agitation afin d'obtenir leur dissolution complète. L'ensemble est maintenu à 75°C sous agitation dans une cuve à double paroi jusqu'à l'étape d'homogénéisation. La base de lait infantile subit ensuite une homogénéisation double effet à 200 / 40 bars, i.e., une première étape d'homogénéisation est réalisée sous une pression de 200 bars et la deuxième étape d'homogénéisation est réalisée sous une pression de 40 bars. La base de lait infantile homogénéisée est ensuite pasteurisée par traitement thermique à environ 80°C pendant 1 à 2 minutes dans le but d'éliminer les risques bactériologiques, en particulier ceux liés à *Cronobacter Sakazakii.*
La base de lait infantile pasteurisée subie ensuite une étape d'atomisation réalisée sous une pression de 160 bars qui permet d'obtenir des gouttelettes d'un diamètre suffisamment faible pour être séchée avec de l'air dont la température à l'entrée dans la chambre est de 183°C et la température à la sortie de la chambre est de 94°C.

Le procédé mis en oeuvre permet ici d'obtenir un débit de 1000 et 2000 kg de poudre/heure.
Le lait liquide reconstitué (prêt à la consommation) obtenu à partir de cette poudre de lait infantile a un extrait sec d'environ 13% dans le biberon. La viscosité de ce lait reconstitué, mesurée à 60 rpm (rotations par minute), à 37°C, est comprise entre 25-45 cP (mobile S61) à un pH proche de la neutralité et comprise entre 130 et 225 cP pour une gamme de pH allant de 5,5 à 3,5. Le lait liquide reconstitué contient 0,52% de pectines et 0,091% de xanthane dans le biberon.

### EXEMPLE 3 :

### Procédé de fabrication d'un lait infantile pasteurisé sous forme de poudre comprenant au moins i) une pectine amidée et faiblement estérifiée, ii) une pectine fortement estérifiée et iii) du xanthane :

La base de lait infantile comprenant 35% d'extrait sec est préparée en mélangeant de l'eau préalablement chauffée à 75°C avec les différents ingrédients du lait infantile (protéines, pectines amidées et faiblement estérifiées, pectines fortement estérifiées, glucides, xanthane, minéraux matières grasses végétales, vitamines et facteurs de croissance). Les pectines sont incorporées dans la base de lait infantile maintenue sous agitation afin d'obtenir leur dissolution complète. L'ensemble est maintenu à 75°C sous agitation dans une cuve à double paroi jusqu'à l'étape d'homogénéisation. La base de lait infantile subit ensuite une homogénéisation double effet à 200 / 40 bars, i.e., une première étape d'homogénéisation est réalisée sous une pression de 200 bars et la deuxième étape d'homogénéisation est réalisée sous une pression de 40 bars. La base de lait infantile homogénéisée est ensuite pasteurisée par traitement thermique à environ 80°C pendant 1 à 2 minutes dans le but d'éliminer les risques bactériologiques, en particulier ceux liés à *Cronobacter Sakazakii.*
La base de lait infantile pasteurisée subie ensuite une étape d'atomisation réalisée sous une pression de 150 bars qui permet d'obtenir des gouttelettes d'un diamètre suffisamment faible pour être séchée avec de l'air dont la température à l'entrée dans la chambre est de 185°C et la température à la sortie de la chambre est de 96°C.
Le procédé mis en oeuvre permet ici d'obtenir un débit de 1000 et 2000 kg de poudre/heure.

Le lait liquide reconstitué (prêt à la consommation) obtenu à partir de cette poudre de lait infantile a un extrait sec d'environ 13% dans le biberon. La viscosité de ce lait reconstitué, mesurée à 60 rpm (rotations par minute), à 37°C, est comprise entre 25-45 cP (mobile S61) à un pH proche de la neutralité et comprise entre 150 et 250 cP pour une gamme de pH allant de 5,8 à 3,5. La poudre de lait infantile obtenue contient 5% de pectines et 0,7% de xanthane. Le lait liquide reconstitué dans le biberon contient donc environ 0,65% de pectines et 0,091% de xanthane.

### EXEMPLE 4 : Comparaison de la viscosité d'un lait reconstitué selon l'invention à la viscosité d'un lait épaissi par adjonction de Gelopectose®

"Gelopectose ®" est un ADDFMS (Aliment Diététique Destiné à des Fins Médicales Spéciales), principalement composé de pectine. La composition exacte est la suivante: pectine (E440), cellulose (E460), Silice (E551), Maltodextrine, Chlorure de sodium, Chlorure de calcium, arôme citron.

La posologie et le mode de préparation exacts donnés par le fabricant sont rappelés ci-dessous :
« Posologie: Utiliser Gelopectose® à la dose de 3 à 5% (soit 3 à 5 g pour 100 ml d'eau avant reconstitution). Pour 90 ml d'eau avant reconstitution, utiliser 2 cuillerées à café rases de Gelopectose (voir mémo- posologique pour le nombre de cuillerée à café de Gelopectose en fonction des différents volumes d'eau).
**Mémo Posologique** :
   Eau : Gelopectose.
   90 ml : 2 c. à café.
   120 ml : 2-3 c. à café.
   150 ml : 3 c. à café.
   180 ml : 3-4 c. à café.
   210 ml : 4-5 c. à café.
   240 ml : 5 c. à café.
   270 ml : 5-6 c. à café.
   300 ml : 6 c. à café.

### Mode d'administration :

- dans un biberon de lait reconstitué très chaud (50 à 60°C), verser la quantité prescrite de Gelopectose. Agiter vigoureusement environ 30 secondes, puis laisser reposer jusqu'à l'obtention du gel et de la température souhaitée pour l'administrer au nourrisson.
- Surtout, ne pas réagiter le biberon après l'obtention du gel.
- Utiliser une eau faiblement minéralisée, conseillée pour la préparation des biberons. Le biberon peut être conservé au réfrigérateur (+ 4°C) 24 heures maximum, pour l'utilisation, réchauffer le au bain-marie sans l'agiter. »

L'objectif de l'étude est de comparer la viscosité d'un lait infantile anti-régurgitation (comprenant un hydrolysat de protéines sériques peu hydrolysées) réalisé selon la présente invention et d'un lait infantile hypoallergénique (HA) (comprenant également un hydrolysat de protéines sériques peu hydrolysées) auquel a été ajouté le produit "Gelopectose®".

### Méthode et outils:

Les deux produits sont reconstitués, dans un bécher, par mélange à de l'eau à 37°C. Le taux de reconstitution est de 13%, c'est-à-dire 13g de poudre dans 90 ml d'eau. 300 ml de chaque solution sont préparés.

Le produit HA est reconstitué en premier, puis chauffé à 60°C comme indiqué par le fabricant du Gelopectose®. Six cuillères à café de Gelopectose® sont ensuite ajoutées au 300 ml de lait présent dans le biberon.
Une forte agitation du biberon est nécessaire pour dissoudre correctement le biberon et empêcher la formation de grumeaux. Le biberon est ensuite refroidi à 37°C.

Le lait infantile anti-régurgitation selon l'invention est reconstitué directement par mélange à de l'eau à 37°C.

Les viscosités des deux produits sont ensuite mesurées à l'aide d'un viscosimètre Brookfield (DV-I Prime) au pH de reconstitution (proche de la neutralité) avec un mobile S61 à 60 rpm (rotations par minute) et à une température de 37°C.

Puis de l'acide chlorhydrique, de molarité égale à 1 (HCl 1 M), est ajouté aux deux produits reconstitués pour atteindre un pH de 3,5. Les viscosités des deux produits acidifiés sont alors mesurées à 37°C et à une vitesse de 60 rpm. Un mobile S62 a été utilisé pour mesurer celle du lait infantile selon l'invention et un mobile S61 pour mesurer celle du lait HA épaissi avec du Gelopectose®. En effet, le lait infantile réalisé selon la présente invention ayant une viscosité importante (cf. résultats ci-dessous) à pH acide, il a fallu changer de mobile pour pouvoir réaliser la mesure.
Les mesures obtenues apparaissent dans le tableau 2 ci-dessous.

**Tableau 2 :**

| | **Viscosités en cP** | |
|---|---|---|
| | **Invention** | **HA avec "Gelopectose®"** |
| **pH= environ 7** | 44 | 90 |
| **pH= 3,5** | 225 | 60 |

### Conclusion

Le lait infantile anti-régurgitation selon la présente invention démontre un bien meilleur comportement, en tant que formule anti-régurgitation, que la formule HA épaissie avec le produit Gelopectose®".

En effet, nous pouvons voir que la viscosité du lait infantile réalisé selon la présente invention augmente à pH acide alors que la viscosité du produit HA avec Gelopectose® diminue pour atteindre une faible viscosité (voir Figure 1).

De plus, le lait infantile réalisé selon la présente invention est plus simple et rapide à utiliser car il ne nécessite pas de chauffer le biberon à 60°C et de le laisser refroidir avant de le donner au bébé.
Notons par ailleurs que cette nécessité de chauffer le lait infantile reconstitué avant d'ajouter le Gelopectose entraîne une destruction des vitamines. Cette destruction est évitée avec le lait infantile selon l'invention qui nécessite simplement d'être dilué, à 37°C, dans l'eau.

Enfin, le lait infantile réalisé selon la présente invention montre une viscosité faible à un pH proche de la neutralité qui facilite la tétée du bébé, alors qu'à l'inverse le lait épaissi à l'aide de Gelopectose® est déjà très visqueux à pH neutre et donc difficilement buvable par le bébé.

### EXEMPLE 5 : Comparaison de la viscosité d'un lait reconstitué comprenant un lait sous forme de poudre selon l'invention obtenu par mélange à sec à la viscosité d'un lait AR classique

Une base de lait infantile comme décrite dans la partie « Base infantile » de la présente invention est réalisée et recueillie sous forme de poudre en sortie de tour de séchage. A cette base poudre sont ensuite ajoutés par mélange à sec, 4% de pectine amidée faiblement estérifiée, 1% de pectine fortement estérifiée et 0,7% de xanthane.

### Méthode et outils:

Le lait infantile liquide est alors réalisé, par dilution du lait infantile sous forme de poudre selon l'invention dans de l'eau chaude (à 60°C) à 13 % d'extrait sec. Ledit lait infantile liquide est ensuite refroidi à 37°C.
Le lait AR classique à base d'amidon est aussi préparé par dilution de la poudre dans de l'eau (également à 37°C) à 13% d'extrait sec.

Les viscosités des deux produits sont ensuite mesurées à l'aide d'un viscosimètre Brookfield (DV-I Prime) au pH de reconstitution (proche de la neutralité) avec un mobile S61 à 60 rpm (rotations par minute) et à une température de 37°C.

De l'acide chlorhydrique, de molarité égale à 1 (HCl 1 M), est ajouté aux deux produits reconstitués pour atteindre un pH de 5,5. Les viscosités des deux produits acidifiés sont alors mesurées à 37°C et à une vitesse de 60 rpm.

Puis de l'acide chlorhydrique, de molarité égale à 1 (HCl 1 M), est de nouveau ajouté aux deux produits reconstitués pour atteindre un pH de 3,5. Les viscosités des deux produits acidifiés sont alors de nouveau mesurées à 37°C et à une vitesse de 60 rpm.

**Tableau 3 :**

| | **Viscosités en cP** | | |
|---|---|---|---|
| **Produits AR** | ***pH neutre*** | ***pH*=*5,5*** | ***pH=3,5*** |
| AR Classique (protéines natives) | 10 | 96 | 241 |
| Lait infantile selon l'invention obtenu en mélange à sec (protéines natives) | 24 | 236 | 234 |

### EXEMPLE 6 : Comparaison de la viscosité d'un lait reconstitué à partir d'un lait sous forme de poudre selon l'invention comprenant au moins une pectine faiblement estérifiée et un des épaississants choisis parmi le xanthane, la Methyle Cellulose, la Carboxy-Methyle Cellulose, l'Hydroxypropyle Cellulose, l'Hydroxy-propyle methyle cellulose et la guar (compositions 1 à 8) et de laits reconstitués à partir de laits sous forme de poudre comprenant de la caroube dite native (compositions 9 et 10) ou de la caroube dite soluble à froid (composition 11).

L'objectif de l'étude est de comparer la viscosité de laits infantiles anti-régurgitation comprenant un hydrolysat de protéines sériques peu hydrolysées réalisés selon la présente invention (compositions 1 à 8) et de laits infantiles comprenant également un hydrolysat de protéines sériques peu hydrolysées et de la caroube dite native (compositions 9 et 10) ou de la caroube soluble à froid (composition 11).

### Méthode et outils:

Les produits selon l'invention sont réalisés sans ajout à sec (compositions 1 à 9). La composition 10 est réalisée selon l'exemple 2 de la demande de brevet FR 2 913 857.
La composition 11 est réalisée par séchage d'une base de lait infantile et ajout à sec de 4% de caroube soluble à froid.

Tous les produits sont reconstitués, dans un bécher, par mélange à de l'eau à 37°C. Le taux de reconstitution est de 13%, c'est-à-dire 13g de poudre dans 90 ml d'eau. 300 ml de chaque solution sont préparés.
Les viscosités des produits sont ensuite mesurées à l'aide d'un viscosimètre Brookfield (DV-I Prime) au pH de reconstitution (proche de la neutralité) avec un mobile S61 (faibles viscosités) à 60 rpm (rotations par minute) et à une température de 37°C.

Puis de l'acide chlorhydrique, de molarité égale à 1 (HCl 1 M), est ajouté aux produits reconstitués pour atteindre un pH de 3,5. Les viscosités des produits acidifiés sont alors mesurées à 37°C et à une vitesse de 60 rpm. Un mobile S62 (hautes viscosités) a été utilisé pour mesurer celle des laits infantiles selon l'invention et de la composition 11 et un mobile S61 (faibles viscosités) pour mesurer celle des laits de composition 9 et 10.

### Compositions:

| | | **Composition 1 selon l'invention** | **Composition 2 selon l'invention** |
|---|---|---|---|
| | | Pour 100 g de poudre | Pour 100 g de poudre |
| Protéines | g | 12,1 | 12,1 |
| Lipides dont: | g | 26,2 | 26,2 |
| - Acide Linoléique | g | 4,5 | 4,5 |
| - Acide a-linolénique | mg | 450 | 450 |
| Glucides dont: | g | 52,7 | 52,7 |
| - Maltodextrines | g | 46,85 | 44,75 |
| - Lactose | g | 0 | 0 |
| - Amidon | g | 0,94 | 0,94 |
| Pectine HM | g | 1,00 | 3,00 |
| Pectine amidée LM | g | 4,00 | 4,00 |
| Xanthane | g | 0,70 | |
| Methyl Cellulose | g | | 0,80 |
| Energie | kcal | 495,90 | 495,90 |
| Minéraux dont : | g | 4,4 | 4,4 |
| - Sodium | mg | 230 | 230 |
| - Potassium | mg | 610 | 610 |
| - Chlore | mg | 340 | 340 |
| - Calcium | mg | 620 | 620 |
| - Phosphore | mg | 340 | 340 |
| - Magnésium | mg | 50 | 50 |
| - Fer | mg | 6 | 6 |
| - Zinc | mg | 4 | 4 |
| - Iode | µg | 70 | 70 |
| - Cuivre | µg | 350 | 350 |
| - Manganèse | µg | 50 | 50 |
| - Sélénium | µg | 10 | 10 |
| - Molybdène | µg | <45 | <45 |
| - Chrome | µg | <45 | <45 |
| - Fluor | µg | <980 | <980 |
| Mélange de vitamines | g | 0,3 | 0,3 |
| | | | |

| | | **Composition 3 selon l'invention** | **Composition 4 selon l'invention** |
|---|---|---|---|
| | | Pour 100 g de poudre | Pour 100 g de poudre |
| Protéines | g | 12,1 | 12,1 |
| Lipides dont: | g | 26,2 | 26,2 |
| - Acide Linoléique | g | 4,5 | 4,5 |
| - Acide a-linolénique | mg | 450 | 450 |
| Glucides dont : | g | 52,7 | 52,7 |
| - Maltodextrines | g | 46,35 | 44,05 |
| - Lactose | g | 0 | 0 |
| - Amidon | g | 0,94 | 0,94 |
| Pectine HM | g | 1,0 | 2,00 |
| Pectine amidée LM | g | 4,20 | 4,0 |
| Carboxy Methyl Cellulose | g | 1,00 | |
| HydroxyPropyl Methyl Cellulose | g | | 2,50 |
| Energie | kcal | 495,90 | 495,90 |
| Minéraux dont : | g | 4,4 | 4,4 |
| - Sodium | mg | 230 | 230 |
| - Potassium | mg | 610 | 610 |
| - Chlore | mg | 340 | 340 |
| - Calcium | mg | 620 | 620 |
| - Phosphore | mg | 340 | 340 |
| - Magnésium | mg | 50 | 50 |
| - Fer | mg | 6 | 6 |
| - Zinc | mg | 4 | 4 |
| - Iode | µg | 70 | 70 |
| - Cuivre | µg | 350 | 350 |
| - Manganèse | µg | 50 | 50 |
| - Sélénium | µg | 10 | 10 |
| - Molybdène | µg | <45 | <45 |
| - Chrome | µg | <45 | <45 |
| - Fluor | µg | <980 | <980 |
| Mélange de vitamines | g | 0,3 | 0,3 |
| | | | |

| | | **Composition 5 selon l'invention** | **Composition 6 selon l'invention** |
|---|---|---|---|
| | | Pour 100 g de poudre | Pour 100 g de poudre |
| Protéines | g | 12,1 | 12,1 |
| Lipides dont: | g | 26,2 | 26,2 |
| - Acide Linoléique | g | 4,5 | 4,5 |
| - Acide a-linolénique | mg | 450 | 450 |
| Glucides dont: | g | 52,7 | 52,7 |
| - Maltodextrines | g | 44,55 | 45,05 |
| - Lactose | g | 0 | 0 |
| - Amidon | g | 0,94 | 0,94 |
| Pectine HM | g | 2,00 | 2,00 |
| Pectine amidée LM | g | 4,0 | 4,0 |
| HydroxyPropyl Cellulose | g | 2,00 | |
| Guar | g | | 1,00 |
| Energie | kcal | 495,90 | 495,9 |
| Minéraux dont : | g | 4,4 | 4,4 |
| - Sodium | mg | 230 | 230 |
| - Potassium | mg | 610 | 610 |
| - Chlore | mg | 340 | 340 |
| - Calcium | mg | 620 | 620 |
| - Phosphore | mg | 340 | 340 |
| - Magnésium | mg | 50 | 50 |
| - Fer | mg | 6 | 6 |
| - Zinc | mg | 4 | 4 |
| - Iode | µg | 70 | 70 |
| - Cuivre | µg | 350 | 350 |
| - Manganèse | µg | 50 | 50 |
| - Sélénium | µg | 10 | 10 |
| - Molybdène | µg | <45 | <45 |
| - Chrome | µg | <45 | <45 |
| - Fluor | µg | <980 | <980 |
| Mélange de vitamines | g | 0,3 | 0,3 |
| | | | |

| | | **Composition 7 selon l'invention** | **Composition 8 selon l'invention** |
|---|---|---|---|
| | | Pour 100 g de poudre | Pour 100 g de poudre |
| Protéines | g | 12,1 | 12,1 |
| Lipides dont : | g | 26,2 | 26,2 |
| - Acide Linoléique | g | 4,5 | 4,5 |
| - Acide a-linolénique | mg | 450 | 450 |
| Glucides dont: | g | 52,7 | 52,7 |
| - Maltodextrines | g | 44,55 | 45,05 |
| - Lactose | g | 0 | 0 |
| - Amidon | g | 0,94 | 0,94 |
| Pectine HM | g | | 2,00 |
| Pectine LM | g | 4,0 | 4,0 |
| Xanthane | g | 1,00 | 1,00 |
| Energie | kcal | 495,90 | 495,9 |
| Minéraux dont : | g | 4,4 | 4,4 |
| - Sodium | mg | 230 | 230 |
| - Potassium | mg | 610 | 610 |
| - Chlore | mg | 340 | 340 |
| - Calcium | mg | 620 | 620 |
| - Phosphore | mg | 340 | 340 |
| - Magnésium | mg | 50 | 50 |
| - Fer | mg | 6 | 6 |
| - Zinc | mg | 4 | 4 |
| - Iode | µg | 70 | 70 |
| - Cuivre | µg | 350 | 350 |
| - Manganèse | µg | 50 | 50 |
| - Sélénium | µg | 10 | 10 |
| - Molybdène | µg | <45 | <45 |
| - Chrome | µg | <45 | <45 |
| - Fluor | µg | <980 | <980 |
| Mélange de vitamines | g | 0,3 | 0,3 |

| | | **Composition 9** | **Composition 10 (selon l'exemple 2 de la demande de brevet** FR 2913 857**)** |
|---|---|---|---|
| | | Pour 100 g de poudre | Pour 100 g de poudre |
| Protéines | g | 12,1 | 12,1 |
| Lipides dont: | g | 26,2 | 26,2 |
| - Acide Linoléique | g | 4,5 | 4,5 |
| - Acide a-linolénique | mg | 450 | 450 |
| Glucides dont : | g | 52,7 | 52,7 |
| - Maltodextrines | g | 44,55 | 46,05 |
| - Lactose | g | 0 | 0 |
| - Amidon | g | 0,94 | 0,94 |
| Pectine HM | g | 2,00 | |
| Pectine amidée LM | g | 4,0 | |
| Gomme de Caroube native | | 1,50 | 4,00 |
| Energie | kcal | 495,9 | 495,9 |
| Minéraux dont : | g | 4,4 | 4,4 |
| - Sodium | mg | 230 | 230 |
| - Potassium | mg | 610 | 610 |
| - Chlore | mg | 340 | 340 |
| - Calcium | mg | 620 | 620 |
| - Phosphore | mg | 340 | 340 |
| - Magnésium | mg | 50 | 50 |
| - Fer | mg | 6 | 6 |
| - Zinc | mg | 4 | 4 |
| - Iode | µg | 70 | 70 |
| - Cuivre | µg | 350 | 350 |
| - Manganèse | µg | 50 | 50 |
| - Sélénium | µg | 10 | 10 |
| - Molybdène | µg | <45 | <45 |
| - Chrome | µg | <45 | <45 |
| - Fluor | µg | <980 | <980 |
| Mélange de vitamines | g | 0,3 | 0,3 |

| | | **Composition 11** | |
|---|---|---|---|
| | | Pour 100 g de poudre | |
| Protéines | g | 12,1 | |
| Lipides dont: | g | 26,2 | |
| - Acide Linoléique | g | 4,5 | |
| - Acide a-linolénique | mg | 450 | |
| Glucides dont: | g | 52,7 | |
| - Maltodextrines | g | 46,05 | |
| - Lactose | g | 0 | |
| - Amidon | g | 0,94 | |
| Gomme de Caroube soluble à froid | g | 4,00 | |
| Energie | kcal | 495,9 | |
| Minéraux dont : | g | 4,4 | |
| - Sodium | mg | 230 | |
| - Potassium | mg | 610 | |
| - Chlore | mg | 340 | |
| - Calcium | mg | 620 | |
| - Phosphore | mg | 340 | |
| - Magnésium | mg | 50 | |
| - Fer | mg | 6 | |
| - Zinc | mg | 4 | |
| - Iode | µg | 70 | |
| - Cuivre | µg | 350 | |
| - Manganèse | µg | 50 | |
| - Sélénium | µg | 10 | |
| - Molybdène | µg | <45 | |
| - Chrome | µg | <45 | |
| - Fluor | µg | <980 | |
| Mélange de vitamines | g | 0,3 | |

### Résultats :

| | **Viscosités en cP** | | | | | | | | | | |
|---|---|---|---|---|---|---|---|---|---|---|---|
| **Compositions** | **1** | **2** | **3** | **4** | **5** | **6** | **7** | **8** | **9** | **10** | **11** |
| **pH = 6,7** | 44 | 24 | 29 | 17 | 38 | 40 | 50 | 48 | 60 | 4 | 74 |
| **pH= 3,5** | 225 | 162 | 195 | 158 | 174 | 155 | 152 | 165 | 17 | 25 | 200 |

### Conclusion:

Les laits infantiles anti-régurgitation selon la présente invention (compositions 1 à 8) démontrent un bien meilleur comportement, en tant que formule anti-régurgitation, que les produits de composition 9 et 10. Le produit de composition 11 donne de bons résultats mais présente l'inconvénient de devoir ajouter au reste de la base infantile, de la caroube soluble à froid en mélange à sec, une telle opération augmentant de manière significative le risque microbiologique.

Ainsi, nous pouvons voir que la viscosité des laits infantiles réalisés selon la présente invention (compositions 1 à 8) augmente à pH acide alors que la viscosité des produits de composition 9 et 10 diminue et les produits reconstitués déphasent rapidement (cf. viscosité des compositions 9 et 10 à pH 3,5). Ces compositions ne conviennent donc pas à une utilisation pour la diminution des régurgitations et/ou du reflux du nourrisson et ne peuvent être utilisées en tant que composition anti-régurgitation et/ou anti-reflux au sens de la présente invention.

## Revendications

1. Méthode de préparation d'un lait infantile anti-reflux et/ou anti-régurgitation sous forme de poudre comprenant les étapes de :
a) préparation d'une base liquide de lait infantile dont la teneur en matière sèche est d'au moins 20% en masse, par mélange, sous agitation, à une température d'au moins 60°C, des éléments constitutifs de ladite base liquide, lesdits éléments comprenant i) au moins une pectine amidée et faiblement estérifiée, ii) au moins un épaississant et/ou gélifiant choisi parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, et la gomme de guar, et éventuellement iii) au moins une pectine fortement estérifiée,
b) homogénéisation de la base liquide obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) séchage par atomisation du mélange obtenu à l'issue de l'étape b), et
d) récupération du lait infantile anti-reflux et/ou anti-régurgitation obtenue à l'issue de l'étape c) sous forme de poudre.

2. Méthode de préparation d'un lait infantile anti-reflux et/ou anti-régurgitation sous forme de poudre selon la revendication 1, comprenant les étapes de :
a) préparation d'une base liquide de lait infantile dont la teneur en matière sèche est d'au moins 20% en masse, par mélange, sous agitation, à une température d'au moins 60°C, des éléments constitutifs de ladite composition,
b) homogénéisation de la base liquide obtenue à l'issue de l'étape a) par fractionnement des éléments constitutifs lors d'une première étape i) réalisée sous une pression comprise entre 100 et 300 bars et lors d'une étape ii) réalisée sous une pression comprise entre 10 et 60 bars,
c) application, à la base liquide obtenue à l'issue de l'étape a) ou à l'issue de l'étape b) d'un traitement thermique à une température comprise entre 60°C et 110°C pendant une durée suffisante pour pasteuriser ladite base,
d) séchage par atomisation du mélange obtenu à l'issue de l'étape c), et
e) récupération du lait infantile anti-reflux et/ou anti-régurgitation pasteurisé obtenu à l'issue de l'étape d) sous forme de poudre.

3. Méthode selon l'une quelconque des revendications précédentes, **caractérisée en ce que** la teneur en matière sèche de la base liquide est d'au moins 35% en masse.

4. Méthode selon l'une quelconque des revendications précédentes, **caractérisée en ce que** l'étape a) de préparation de la base liquide est réalisée sous agitation à une température comprise entre environ 60°C et environ 90°C, et est maintenue sous agitation jusqu'à l'étape d'homogénéisation.

5. Méthode selon l'une quelconque des revendications précédentes, **caractérisée en ce que** l'étape i) d'homogénéisation est réalisée sous une pression comprise entre environ 170 bars et environ 200 bars et l'étape ii) d'homogénéisation est réalisée sous une pression comprise entre environ 30 bars et environ 40 bars.

6. Méthode selon la revendication 5, **caractérisée en ce que** les éléments constitutifs du lait infantile comprennent en outre de l'amidon précuit et/ou prégélatinisé.

7. Méthode selon la revendication 6, **caractérisée en ce que** le lait infantile comprend une fraction protéique contenant une majorité de protéines hydrolysées.

8. Lait infantile anti-reflux et/ou anti-régurgitation sous forme de poudre susceptible d'être obtenu à l'aide d'une méthode selon l'une des revendications 1 à 7.

9. lait infantile sous forme de poudre selon la revendication 8, **caractérisé en ce qu'**il comprend au moins 94 % de matières sèches.

10. Lait infantile anti reflux et/ou anti-régurgitation sous forme de poudre comprenant au moins une pectine amidée et faiblement estérifiée, et au moins un épaississant et/ou gélifiant choisi parmi la gomme xanthane, la carboxy-méthyle cellulose, l'hydroxy-propyle cellulose, l'hydroxy-méthyle cellulose, la méthyle cellulose, l'hydroxy-propyle méthyle cellulose, et la gomme de guar.

11. Lait infantile anti reflux et/ou anti-régurgitation selon la revendication 10, **caractérisé en ce que** sa fraction protéique contient une majorité de protéines hydrolysées et/ou d'acides aminés en mélange.

12. Lait infantile anti reflux et/ou anti-régurgitation selon la revendication 10 ou 11, **caractérisé en ce qu'**il comprend en outre au moins une pectine fortement estérifiée.

13. Lait infantile anti reflux et/ou anti-régurgitation selon l'une des revendications 10 à 12, **caractérisé en ce qu'**il comprend pour 100 grammes de matière sèche, 0,5 à 10 grammes de pectine et 0,5 à 4 grammes de l'épaississant choisi.

14. Lait infantile anti reflux et/ou anti-régurgitation selon la revendication 13, **caractérisé en ce que** l'épaississant choisi est le xanthane.

15. Lait infantile reconstitué à partir de la composition sous forme de poudre selon l'une quelconque des revendications 10 à 14, **caractérisé en ce qu'**il est liquide à pH7 et visqueux à un pH compris entre 6 et 3,5, en particulier à un pH compris entre 5,8 et 5.

16. Lait infantile reconstitué selon la revendication 15, **caractérisé en ce que** la viscosité dudit lait, à un pH compris entre 5,5 et 3,5, est comprise entre 200 mPa.s et 300 mPa.s.

## Patentansprüche

1. Verfahren zur Herstellung einer Anti-Regurgitation- und/oder Anti-Reflux-Säuglingsmilch in Pulverform, umfassend die Schritte:
a) Herstellung einer flüssigen Säuglingsmilch-Basis, deren Trockenmassegehalt wenigstens 20 Gew.-% beträgt, durch Mischung der konstitutiven Bestandteile besagter flüssiger Basis unter Rühren bei einer Temperatur von wenigstens 60°C, wobei besagte Bestandteile umfassen: i) wenigstens ein schwach verestertes und amidiertes Pektin, ii) wenigstens ein Verdickungsmittel und/oder einen Gelbildner, ausgewählt aus Xanthan-Gummi, Carboxymethylcellulose, Hydroxypropylcellulose, Hydroxymethylcellulose, Methylcellulose, Hydroxypropylmethylcellulose und Guar-Gummi, und gegebenenfalls iii) wenigstens ein stark verestertes Pektin,
b) Homogenisation der in Schritt a) erhaltenen flüssigen Basis durch Fraktionierung der konstitutiven Bestandteile bei einem ersten Schritt i), der unter einem Druck zwischen 100 und 300 bar durchgeführt wird, und bei einem Schritt ii), der unter einem Druck zwischen 10 und 60 bar durchgeführt wird,
c) Sprühtrocknung des in Schritt b) erhaltenen Gemisches, und
d) Wiedergewinnung der in Schritt c) in Pulverform erhaltenen Anti-Regurgitation- und/oder Anti-Reflux-Säuglingsmilch.

2. Verfahren zur Herstellung einer Anti-Regurgitation- und/oder Anti-Reflux-Säuglingsmilch in Pulverform gemäß Anspruch 1, umfassend die Schritte:
a) Herstellung einer flüssigen Säuglingsmilch-Basis, deren Trockenmassegehalt wenigstens 20 Gew.-% beträgt, durch Mischung der konstitutiven Bestandteile besagter Zusammensetzung unter Rühren bei einer Temperatur von wenigstens 60°C,
b) Homogenisation der in Schritt a) erhaltenen flüssigen Basis durch Fraktionierung der konstitutiven Bestandteile bei einem ersten Schritt i), der unter einem Druck zwischen 100 und 300 bar durchgeführt wird, und bei einem Schritt ii), der unter einem Druck zwischen 10 und 60 bar durchgeführt wird,
c) Unterziehung der in Schritt a) oder in Schritt b) erhaltenen flüssigen Basis einer thermischen Behandlung bei einer Temperatur zwischen 60°C und 110°C und einer für eine Pasteurisierung besagter Basis hinreichenden Dauer,
d) Sprühtrocknung des in Schritt c) erhaltenen Gemisches, und
e) Wiedergewinnung der in Schritt d) in Pulverform erhaltenen pasteurisierten Anti-Regurgitation- und/oder Anti-Reflux-Säuglingsmilch.

3. Verfahren gemäß einem der vorhergehenden Ansprüche, **dadurch gekennzeichnet, dass** der Trockenmassegehalt der flüssigen Basis wenigstens 35 Gew.-% beträgt.

4. Verfahren gemäß einem der vorhergehenden Ansprüche, **dadurch gekennzeichnet, dass** der Herstellungsschritt a) der flüssigen Basis unter Rühren bei einer Temperatur zwischen ungefähr 60°C und ungefähr 90°C durchgeführt wird und bis zum Homogenisationsschritt unter Rühren aufrechterhalten wird.

5. Verfahren gemäß einem der vorhergehenden Ansprüche, **dadurch gekennzeichnet, dass** der Homogenisationsschritt i) unter einem Druck zwischen ungefähr 170 bar und ungefähr 200 bar durchgeführt wird und der Homogenisationsschritt ii) unter einem Druck zwischen ungefähr 30 bar und ungefähr 40 bar durchgeführt wird.

6. Verfahren gemäß Anspruch 5, **dadurch gekennzeichnet, dass** die konstitutiven Bestandteile der Säuglingsmilch außerdem vorgekochte und/oder vorgelatinierte Stärke umfassen.

7. Verfahren gemäß Anspruch 6, **dadurch gekennzeichnet, dass** die Säuglingsmilch eine Proteinfraktion umfasst, die überwiegend hydrolysierte Proteine enthält.

8. Anti-Regurgitation- und/oder Anti-Reflux-Säuglingsmilch in Pulverform, die mithilfe eines Verfahrens gemäß einem der Ansprüche 1 bis 7 erhalten werden kann.

9. Säuglingsmilch in Puverform gemäß Anspruch 8, **dadurch gekennzeichnet, dass** sie wenigstens 94% Trockenmasse umfasst.

10. Anti-Regurgitation- und/oder Anti-Reflux-Säuglingsmilch in Pulverform, umfassend wenigstens ein schwach verestertes und amidiertes Pektin und wenigstens ein Verdickungsmittel und/oder einen Gelbildner, ausgewählt aus Xanthan-Gummi, Carboxymethylcellulose, Hydroxypropylcellulose, Hydroxymethylcellulose, Methylcellulose, Hydroxypropylmethylcellulose und Guar-Gummi.

11. Anti-Regurgitation- und/oder Anti-Reflux-Säuglingsmilch gemäß Anspruch 10, **dadurch gekennzeichnet, dass** ihre Proteinfraktion überwiegend hydrolysierte Proteine und/oder Aminosäuren als Gemisch enthält.

12. Anti-Regurgitation- und/oder Anti-Reflux-Säuglingsmilch gemäß Anspruch 10 oder 11, **dadurch gekennzeichnet, dass** sie außerdem wenigstens ein stark verestertes Pektin umfasst.

13. Anti-Regurgitation- und/oder Anti-Reflux-Säuglingsmilch gemäß einem der Ansprüche 10 bis 12, **dadurch gekennzeichnet, dass** sie auf 100 Gramm Trockenmasse 0,5 bis 10 Gramm Pektin und 0,5 bis 4 Gramm ausgewähltes Verdickungsmittel umfasst.

14. Anti-Regurgitation- und/oder Anti-Reflux-Säuglingsmilch gemäß Anspruch 13, **dadurch gekennzeichnet, dass** das ausgewählte Verdickungsmittel Xanthan ist.

15. Säuglingsmilch, rekonstituiert aus der Zusammensetzung in Pulverform gemäß einem der Ansprüche 10 bis 14, **dadurch gekennzeichnet, dass** sie bei pH 7 flüssig und bei einem pH zwischen 6 und 3,5, insbesondere bei einem pH zwischen 5,8 und 5, viskos ist.

16. Rekonstituierte Säuglingsmilch gemäß Anspruch 15, **dadurch gekennzeichnet, dass** die Viskosität besagter Milch bei einem pH zwischen 5,5 und 3,5 zwischen 200 mPa·s s und 300 mPa·s beträgt.

## Claims

1. A method for preparing an anti-reflux and/or anti-regurgitation infant milk as a powder comprising the steps of:
a) preparing a liquid infant milk base, the dry material content of which is of at least 20% by mass, by mixing, with stirring, at a temperature of at least 60°C, constitutive elements of said liquid base, said elements comprising i) at least one amidated and weakly esterified pectin, ii) at least one thickener and/or gelling agent selected from xanthan gum, carboxymethylcellulose, hydroxypropylcellulose, hydroxymethylcellulose, methylcellulose, hydroxypropylmethylcellulose, and guar gum, and optionally iii) at least one highly esterified pectin,
b) homogenizing the obtained liquid base at the end of step a) by fractionation of the constitutive elements during a first step i) carried out under a pressure comprised between 100 and 300 bars and during a step ii) carried out under a pressure comprised between 10 and 60 bars,
c) drying by atomization the mixture obtained at the end of step b), and
d) recovering the anti-reflux and/or anti-regurgitation infant milk obtained at the end of step c) as a powder.

2. The method for preparing an anti-reflux and/or anti-regurgitation infant milk as a powder according to claim 1, comprising the steps of:
a) preparing a liquid infant milk base, the dry material content of which is of at least 20% by mass, by mixing, with strung, at a temperature of at least 60°C, of the constitutive elements of said composition,
b) homogenizing the obtained liquid base at the end of step a) by fractionation of the constitutive elements during a first step i) carried out under a pressure comprised between 100 and 300 bars and during a step ii) carried out under a pressure comprised between 10 and 60 bars,
c) applying to the liquid base obtained at the end of step a) or at the end of step b) a heat treatment at a temperature comprised between 60°C and 110°C for a sufficient time for pasteurizing said base,
d) drying by atomization the mixture obtained at the end of step c), and
e) recovering the anti-reflux and/or anti-regurgitation infant milk obtained at the end of step d) as a powder.

3. The method according to any one of the preceding claims, **characterized in that** the dry material content of the liquid base is of at least 35% by mass.

4. The method according to any one of the preceding claims, **characterized in that** step a) for preparing the liquid base is carried out with stirring at a temperature comprised between about 60°C and about 90°C, and is maintained with stirring until the homogenization step.

5. The method according to any one of the preceding claims, **characterized in that** the homogenization step i) is carried out under a pressure comprised between about 170 bars and about 200 bars and the homogenization step ii) is carried out under a pressure comprised between about 30 bars and about 40 bars.

6. The method according to claim 5, **characterized in that** the constitutive elements of the infant milk further comprise precooked and/or pregelatinized starch.

7. The method according to claim 6, **characterized in that** the infant milk comprises a protein fraction containing a majority of hydrolyzed proteins.

8. An anti-reflux and/or anti-regurgitation infant milk as a powder which may be obtained by means of a method according to one of claims 1 to 7.

9. An infant milk as a powder according to claim 8, **characterized in that** it comprises at least 94% of dry materials.

10. An anti-reflux and/or anti-regurgitation infant milk as a powder comprising at least one amidated and weakly esterified pectin, and at least a one thickener and/or gelling agent selected from xanthan gum, carboxymethylcellulose, hydroxypropylcellulose, hydroxymethylcellulose, methylcellulose, hydroxypropylmethylcellulose, and guar gum.

11. The anti-reflux and/or anti-regurgitation infant milk according to claim 10, **characterized in that** its protein fraction contains a majority of hydrolyzed proteins and/or amino acids in a mixture.

12. The anti-reflux and/or anti-regurgitation infant milk according to claim 10 or 11, **characterized in that** it further comprises at least one highly esterified pectin.

13. The anti-reflux and/or anti-regurgitation infant milk according to one of claims 10 to 12, **characterized in that** it comprises for 100 grams of dry material, 0.5 to 10 grams of pectin and 0.5 to 4 grams of the selected thickener.

14. The anti-reflux and/or anti-regurgitation infant milk according to claim 13, **characterized in that** the selected thickener is xanthan.

15. A reconstituted infant milk from the composition as a powder according to any one of claims 10 to 14, **characterized in that** it is liquid at pH 7 and viscous at a pH comprised between 6 and 3.5, in particular at a pH comprised between 5.8 and 5.

16. The reconstituted infant milk according to claim 15, **characterized in that** the viscosity of said milk, at a pH comprised between 5.5 and 3.5, is comprised between 200 mPa.s and 300 mPa.s.
